# EUROPÄISCHE PATENTSCHRIFT

(11) **EP 2 704 646 B1**
(45) Veröffentlichungstag und Bekanntmachung des Hinweises auf die Patenterteilung: **19.08.2015**
(21) Anmeldenummer: 12717137.9
(22) Anmeldetag: 30.04.2012
(51) Int. Cl.: A61B 17/16, A61B 17/00, A61C 1/18

(54) **CHIRURGISCHES KUPPLUNGSSYSTEM UND CHIRURGISCHES ANTRIEBSSYSTEM**
SURGICAL COUPLING SYSTEM AND SURGICAL DRIVE SYSTEM
SYSTÈME D'ACCOUPLEMENT CHIRURGICAL ET SYSTÈME D'ENTRAÎNEMENT CHIRURGICAL

(30) Priorität: 06.05.2011 DE 102011050192
(43) Veröffentlichungstag der Anmeldung: 12.03.2014
(73) Patentinhaber: Aesculap AG, 78532 Tuttlingen (DE)
(72) Erfinder: HOEGERLE, Roland, 78532 Tuttlingen (DE); MACHILL, Martin, 78604 Rietheim-Weilheim (DE); PFISTER, Ralf, 78647 Trossingen (DE)
(74) Vertreter: Hoeger, Stellrecht & Partner Patentanwälte mbB
(86) Internationale Anmeldenummer: PCT/EP2012/057918
(87) Internationale Veröffentlichungsnummer: WO 2012/152611

(56) Entgegenhaltungen:
- DE-A1-102006 051 511

## Beschreibung

Die vorliegende Erfindung betrifft ein chirurgisches Kupplungssystem umfassend eine erste chirurgische Kupplungsvorrichtung und eine zweite chirurgische Kupplungsvorrichtung mit jeweils mindestens zwei mechanisch miteinander in Eingriff bringbaren elektrischen Kupplungskontakten, wobei die erste und die zweite Kupplungsvorrichtung in einer Trennstellung vollständig voneinander getrennt sind und in einer mechanischen Kupplungsstellung mechanisch miteinander in Eingriff stehen.

Ferner betrifft die vorliegende Erfindung ein chirurgisches Antriebssystem umfassend mindestens ein chirurgisches Handstück mit einem Antrieb in Form eines Elektromotors und mindestens eine elektrische Versorgungsleitung, welche Versorgungsleitung ein erstes, mit dem mindestens einen Handstück lösbar verbindbares Ende und ein zweites, mit einer Steuer- und/oder Regelungsvorrichtung zum Steuern und/oder Regeln des Elektromotors verbundenes oder lösbar verbindbares Ende aufweist.

Ein chirurgisches Kupplungssystem sowie ein chirurgisches Antriebssystem der eingangs beschriebenen Art sind beispielsweise aus der DE 10 2006 051511 A1 oder der DE 102 25 857 A1 bekannt. Chirurgische Handstücke mit einem integrierten Antrieb in Form eines Elektromotors, nachfolgend auch als chirurgische Motorhandstücke bezeichnet, werden bei dem bekannten Antriebssystem über eine Steckerverbindung mit einer Versorgungsleitung, die auch als Anschlusskabel bezeichnet werden kann, elektrisch gekuppelt. Diese Steckerverbindung erfolgt im sterilen Bereich, um die Handstücke auch intraoperativ wechseln zu können. Dies bedeutet jedoch, dass die Steckerverbindung nach jedem Einsatz aufbereitet, also gereinigt und sterilisiert werden muss. Insbesondere durchlaufen die Steckerverbindungen dabei einen Heißdampfsterilisationprozess.

Es ist bekannt, zum Aktivieren der Handstücke Betätigungselemente vorzusehen, beispielsweise Handschalter an einer der beiden Kupplungsvorrichtungen des Kupplungssystems, beispielsweise an der am Anschlusskabel vorgesehenen Kupplungsvorrichtung, oder Fußschalter. Wird zusätzlich auch noch die Anforderung gestellt, dass die Art des Handstücks automatisch von einer Steuer- und/oder Regelungseinrichtung des Antriebssystems, die nachfolgend auch nur als Motorsteuerung bezeichnet wird, abgefragt werden kann, ist es praktisch nicht möglich, das Anschlusskabel mit nur so vielen Leitungen vorzusehen, wie der Elektromotor Wicklungen aufweist, also bei einem drei Motorwicklungen umfassenden Elektromotor ein nur drei Leitungen umfassendes Anschlusskabel. Zusätzliche Steuer- oder Abfrageleitungen vorzusehen bedeutet aber auch, dass das Kupplungssystem entsprechend mehr elektrische Anschlusskontakte aufweisen muss. Da die Handstücke nach jedem Einsatz wieder aufbereitet werden, ergibt sich bei heutigen Systemen eine unerwünscht hohe Ausfallrate aufgrund elektrischer Kontaktprobleme. Dies nicht zuletzt deshalb, weil auch aktuell im Einsatz befindliche Schaltelemente zur Aktivierung eines Motorstromkreises korrosionsanfällig sind.

Es ist daher eine Aufgabe der vorliegenden Erfindung, chirurgische Kupplungssysteme sowie chirurgische Antriebssysteme der eingangs beschriebenen Art so zu verbessern, dass sie einfacher und sicherer handzuhaben sind.

Diese Aufgabe wird bei einem chirurgischen Kupplungssystem der eingangs beschriebenen Art erfindungsgemäß dadurch gelöst, dass es eine elektrische Schalteinrichtung umfasst, welche in der Kupplungsstellung von einer AUS-Stellung, in welcher mindestens ein erster elektrischer Kupplungskontakt der ersten Kupplungsvorrichtung und mindestens ein erster elektrischer Kupplungskontakt der zweiten Kupplungsvorrichtung außer Eingriff stehen, in eine AN-Stellung, in welcher der mindestens eine erste elektrische Kupplungskontakt der ersten Kupplungsvorrichtung und der mindestens eine erste elektrische Kupplungskontakt der zweiten Kupplungsvorrichtung elektrisch leitend in Kontakt oder Eingriff stehen, bringbar ist und/oder umgekehrt.

Mit dem in der vorgeschlagenen Weise weitergebildeten chirurgischen Kupplungssystem ist es insbesondere möglich, die zusammenwirkenden Kupplungsvorrichtungen als Schalteinrichtung zu nutzen. Mit anderen Worten kann so beispielsweise eine Steckerverbindung zwischen der Versorgungsleitung und dem Handstück gleichzeitig auch als Schalter genutzt werden, welcher es einer Bedienperson ermöglicht, zum Beispiel den Elektromotor des Handstücks bei Bedarf zu aktivieren und auch wieder zu deaktivieren. So kann das Kupplungssystem insgesamt eine Doppelfunktion ausüben. Zum einen kann es eine mechanische Verbindung zwischen dem Handstück und beispielsweise einer Versorgungsleitung herstellen. Zum anderen kann es auch als Schalteinrichtung genutzt werden, um beispielsweise Motorwicklungen eines Elektromotors des Handstücks bei Bedarf bestromen zu können. Durch diese Ausgestaltung ist es insbesondere möglich, die Versorgungsleitung mit nur so vielen Verbindungsleitungen auszustatten, wie der Elektromotor Motorwicklungen umfasst. Damit kann die Zahl der erforderlichen elektrischen Kontaktelemente minimiert werden, insbesondere auf die Zahl der Motorwicklungen des Elektromotors. Auf weitere Leitungen, beispielsweise um bei herkömmlichen Antriebssystemen den Elektromotor zu betätigen und gegebenenfalls dessen Typ oder Bauart abzufragen, kann verzichtet werden.

Günstig ist es, wenn die erste und die zweite Kupplungsvorrichtung jeweils mindestens einen zweiten elektrischen Kupplungskontakt umfassen und wenn die zweiten Kupplungskontakte in der AUS-Stellung und in der AN-Stellung miteinander elektrisch leitend in Kontakt oder Eingriff stehen. Mit anderen Worten ist es so möglich, beispielsweise über die bereits in der AUS-Stellung elektrisch leitend in Kontakt oder Eingriff stehenden zweiten elektrischen Kupplungskontakte vor Aktivierung, also insbesondere vor einer Bestromung, des Elektromotors eine Abfrage spezifischer elektrischer Kenngrößen des Handstücks durchzuführen. Beispielsweise kann in der AUS-Stellung eine Widerstandsmessung vorgenommen werden, über die sich die Art des Handstücks sicher und eindeutig identifizieren lässt. Vorzugsweise sind hierfür jeweils zwei zweite elektrische Kupplungskontakte vorgesehen, wobei die zwei zweiten elektrischen Kupplungskontakte des Handstücks elektrisch leitend miteinander über mindestens eine Motorwicklung verbunden sind.

Besonders vorteilhaft ist es, wenn die eine der beiden Kupplungsvorrichtungen mindestens ein erstes Kupplungselement umfasst, wenn die andere der beiden Kupplungsvorrichtung mindestens ein zweites Kupplungselement umfasst, welches korrespondierend zum mindestens einen ersten Kupplungselement ausgebildet ist, und wenn das mindestens eine erste und das mindestens eine zweite Kupplungselement in der Kupplungsstellung miteinander in Eingriff stehen. Durch die mindestens einen ersten und mindestens einen zweiten Kupplungselemente kann beispielsweise eine mechanische Verbindung der beiden Kupplungsvorrichtungen auf einfache Weise realisiert werden, und zwar insbesondere unabhängig von einem Schaltzustand der Schalteinrichtung. Mit anderen Worten können die Kupplungsvorrichtungen die Kupplungsstellung einnehmen und in dieser wahlweise die AN-Stellung oder AUS-Stellung einnehmen. So können die beiden Kupplungsvorrichtungen mechanisch eine Einheit bilden, und zwar sowohl in der AN-Stellung als auch in der AUS-Stellung.

Besonders einfach wird der Aufbau des Kupplungssystems, wenn das erste Kupplungselement in Form einer Kupplungshülse ausgebildet ist und wenn das zweite Kupplungselement in Form eines in die Kupplungshülse einführbaren Kupplungszapfens ausgebildet ist. Die Kupplungshülse kann entweder an der ersten Kupplungsvorrichtung oder an der zweiten Kupplungsvorrichtung vorgesehen sein. Denkbar ist es auch, den Kupplungszapfen selbst mit einer Ausnehmung zu versehen, in welchen dann ein weiterer, innerhalb der Kupplungshülse angeordneter zweiter Kupplungszapfen eingreifen kann. Auf diese Weise kann eine stabile mechanische Verbindung zwischen den beiden Kupplungsvorrichtungen hergestellt werden.

Gemäß einer weiteren bevorzugten Ausführungsform der Erfindung kann vorgesehen sein, dass das Kupplungssystem eine Schaltstellungssicherungseinrichtung zum mechanischen Sichern der Schalteinrichtung in der AN-Stellung und in der AUS-Stellung umfasst. Werden die beiden Kupplungsvorrichtungen miteinander gekoppelt, so kann durch die Schaltstellungssicherungseinrichtung insbesondere sichergestellt werden, dass die Schalteinrichtung nicht ohne weiteres von der AN-Stellung in die AUS-Stellung und umgekehrt überführt werden kann. Beispielsweise kann die Schalteinrichtung mechanisch sowohl in der AN-Stellung als auch in der AUS-Stellung verriegelt werden. Auf diese Weise kann bei entsprechender Ausgestaltung beispielsweise verhindert werden, dass die beiden Kupplungsvorrichtungen beim Kuppeln direkt die AN-Stellung einnehmen, wodurch der Motor unkontrolliert bestromt werden könnte. Mit anderen Worten können die Kupplungsvorrichtungen mittels der Schaltstellungssicherungseinrichtung vorzugsweise in der Kupplungsstellung zunächst nur die AUS-Stellung einnehmen. Aus der AUS-Stellung muss die Schalteinrichtung dann wiederum gezielt von einer Bedienperson in die AN-Stellung überführt werden.

Günstig ist es, wenn die Schaltstellungssicherungseinrichtung eine Verriegelungseinrichtung zum mechanischen Verriegeln der die Kupplungsstellung einnehmenden ersten und zweiten Kupplungsvorrichtungen in der AN-Stellung und in der AUS-Stellung und eine Löseeinrichtung zum mechanischen Lösen der Verriegelungseinrichtung umfasst. Insbesondere kann die Verriegelungseinrichtung zum automatischen mechanischen Verriegeln der beiden Kupplungsvorrichtungen sowohl in der AN-Stellung als auch in der AUS-Stellung ausgebildet sein. Werden beispielsweise die beiden voneinander getrennten Kupplungsvorrichtungen miteinander in Eingriff gebracht, so ermöglicht es die Verriegelungseinrichtung insbesondere, zunächst die beiden Kupplungsvorrichtungen in der AUS-Stellung mechanisch zu verriegeln. Mit der Löseeinrichtung kann dann beispielsweise die Verriegelungseinrichtung mechanisch durch entsprechende Betätigung der Löseeinrichtung gelöst werden, um die Schalteinrichtung von der AUS-Stellung in die AN-Stellung zu überführen. Die Verriegelungseinrichtung verriegelt vorzugsweise in der AN-Stellung auch automatisch die Kupplungsvorrichtungen mechanisch gegeneinander, so dass eine Rückführung der Schalteinrichtung von der AN-Stellung in die AUS-Stellung nur möglich ist durch eine gezielte Betätigung der Löseeinrichtung, die dann mechanisch die Verriegelungseinrichtung löst, um die Kupplungsvorrichtungen relativ zueinander wieder in die AUS-Stellung zu überführen. Um die Kupplungsvorrichtungen gegebenenfalls wieder voneinander zu trennen, muss ausgehend von der AUS-Stellung die Löseeinrichtung wiederum betätigt werden, um die Verriegelungseinrichtung zu lösen. So kann eine hohe Betriebssicherheit erreicht und ein gezieltes Schalten, das heißt Überführen der Schalteinrichtung von der AN-Stellung in die AUS-Stellung und umgekehrt, erreicht werden.

Vorteilhaft ist es, wenn die Verriegelungseinrichtung erste und zweite Verriegelungselemente sowie ein drittes Verriegelungselement umfasst, wenn die ersten und zweiten Verriegelungselemente an der einen der beiden Kupplungsvorrichtungen angeordnet oder ausgebildet sind, wenn das dritte Verriegelungselement an der anderen der beiden Kupplungsvorrichtungen angeordnet oder ausgebildet ist und wenn das erste und das dritte Verriegelungselement in der AN-Stellung in Eingriff stehen und wenn das zweite und das dritte Verriegelungselement in der AUS-Stellung in Eingriff stehen. Eine Verriegelungseinrichtung mit mindestens den drei genannten Verriegelungselementen vorzusehen ermöglicht es auf einfache Weise, die Kupplungsvorrichtungen in mindestens zwei unterschiedlichen Stellungen relativ zueinander zu verriegeln, nämlich beispielsweise in der AN-Stellung und in der AUS-Stellung.

Besonders einfach herstellen lässt sich die Verriegelungseinrichtung, wenn das erste und das zweite Verriegelungselement in Form erster und zweiter Verriegelungsausnehmungen ausgebildet sind und wenn das dritte Verriegelungselement in Form eines mit der ersten und mit der zweiten Verriegelungsausnehmung in Eingriff bringbaren Verriegelungsvorsprungs ausgebildet ist. Beispielsweise kann der Verriegelungsvorsprung wahlweise in die erste oder in die zweite Verriegelungsausnehmung eingreifen, um so die Kupplungsvorrichtungen relativ zueinander zu verriegeln beziehungsweise zu sichern, beispielsweise in der AN-Stellung und in der AUS-Stellung. Selbstverständlich wäre es auch denkbar, die ersten und zweiten Verriegelungselemente in Form von Verriegelungsvorsprüngen auszubilden und mindestens ein drittes Verriegelungselement in Form einer Verriegelungsausnehmung vorzusehen.

Um den Aufbau des Kupplungssystems weiter zu vereinfachen, ist es günstig, wenn die erste und die zweite Verriegelungsausnehmung in Form axial voneinander beabstandeter Ringnuten ausgebildet sind und wenn der Verriegelungsvorsprung in Form eines in radialer Richtung bewegbar gelagerten Zapfens ausgebildet ist. Beispielsweise können die Ringnuten an der Kupplungshülse oder dem Kupplungszapfen ausgebildet und der bewegbar gelagerte Zapfen in Richtung auf die Längsachse hin oder von der Längsachse weg an einer der beiden Kupplungsvorrichtungen gelagert oder gehalten sein, um wahlweise in die Ringnuten einzugreifen oder diese freizugeben.

Besonders kompakt ausbilden lässt sich das Kupplungssystem, wenn die Verriegelungsausnehmungen in Richtung auf eine Längsachse der Kupplungshülse hin geöffnet sind. Beispielsweise kann so der Verriegelungsvorsprung in Form eines Kupplungszapfens in radialer Richtung bewegbar gelagert sein, um in der AN-Stellung und in der AUS-Stellung jeweils in eine der beiden Verriegelungsausnehmungen einzugreifen.

Vorteilhaft ist es, wenn die Verriegelungseinrichtung eine Vorspanneinrichtung umfasst zum Halten der Verriegelungselemente in der AN-Stellung beziehungsweise in der AUS-Stellung. Durch die Vorspanneinrichtung kann insbesondere sichergestellt werden, dass die in der AN-Stellung beziehungsweise in der AUS-Stellung miteinander in Eingriff stehenden Verriegelungselemente nur entgegen der Wirkung der Vorspanneinrichtung außer Eingriff gebracht werden können. Ferner kann es die Vorspanneinrichtung optional auch ermöglichen, dass beim in Eingriff Bringen der Kupplungsvorrichtungen die Verriegelungselemente automatisch miteinander in Eingriff gebracht werden, sobald die Kupplungsvorrichtungen relativ zueinander derart positioniert sind, dass sie die AN-Stellung beziehungsweise die AUS-Stellung einnehmen können.

Günstig ist es, wenn die Verriegelungselemente nur entgegen einer von der Vorspanneinrichtung ausgeübten vorspannenden Kraft außer Eingriff bringbar sind. Dadurch können die beiden miteinander gekuppelten Kupplungsvorrichtungen nur gezielt durch Betätigen der Löseeinrichtung und entgegen der Wirkung der Vorspanneinrichtung von der AN-Stellung oder der AUS-Stellung in eine andere Stellung überführt werden.

Vorzugsweise sind das erste und das dritte Verriegelungselement und das zweite und das dritte Verriegelungselement in radialer Richtung bezogen auf eine Längsachse des Kupplungssystems relativ zueinander zum in und außer Eingriff Bringen bewegbar angeordnet oder ausgebildet. Können die beiden Kupplungsvorrichtungen beispielsweise in Richtung der Längsachse des Kupplungssystems aufeinander zu bewegt und miteinander in Eingriff gebracht werden, so kann eine Verriegelung beziehungsweise ein Lösen derselben voneinander nur in Folge einer Betätigung in einer Richtung quer zur Längsachse, insbesondere in radialer Richtung, erfolgen. Auf diese Weise kann insbesondere eine Betriebssicherheit des Kupplungssystems erhöht werden.

Vorteilhaft ist es, wenn die Lösevorrichtung ausgebildet ist zum außer Eingriff Bringen der ersten und dritten Verriegelungselemente von der AN-Stellung in eine Schaltstellung, in welcher die Kupplungsvorrichtungen relativ zueinander in axialer Richtung bewegbar sind. Diese Ausgestaltung ermöglicht es, durch Betätigen der Lösevorrichtung die in der AN-Stellung gegen eine Relativbewegung gesicherten Kupplungsvorrichtung in die Schaltstellung zu überführen, in welcher sie relativ zueinander in axialer Richtung bewegbar sind, beispielsweise in Richtung einer vom Kupplungssystem definierten Längsachse.

Ferner kann es vorteilhaft sein, wenn die Löseeinrichtung ausgebildet ist zum außer Eingriff Bringen der zweiten und dritten Verriegelungselemente von der AUS-Stellung in die Schaltstellung. Insbesondere können so durch Betätigung der Lösevorrichtung die beiden Kupplungsvorrichtungen von der AUS-Stellung in die Schaltstellung überführt werden, vorzugsweise durch eine Bewegung in axialer Richtung relativ zueinander. Dies ermöglicht es beispielsweise, die Kupplungsvorrichtungen ausgehend von der AUS-Stellung voneinander zu trennen oder in die AN-Stellung zu bewegen.

Die Handhabbarkeit des Kupplungssystems für eine Bedienperson lässt sich insbesondere dadurch weiter verbessern, dass die Löseeinrichtung ein bewegbar gelagertes Löseglied umfasst, welches von einer unbetätigten Stellung in eine betätigte Stellung bringbar ist und/oder umgekehrt. Insbesondere kann das Löseglied dazu dienen, die in der AN- beziehungsweise AUS-Stellung miteinander in Eingriff stehenden Verriegelungselemente außer Eingriff zu bringen.

Günstig ist es, wenn das Löseglied mit dem dritten Verriegelungselement direkt oder indirekt in Eingriff oder Kontakt bringbar ist zum Überführen der Verriegelungseinrichtung aus der AN-Stellung oder aus der AUS-Stellung in die Schaltstellung. So kann beispielsweise durch einfaches Betätigen des Löseglieds und Überführen desselben von der unbetätigten Stellung in die betätigte Stellung im Zusammenwirken mit dem dritten Verriegelungselement die Verriegelungseinrichtung aus der AN-Stellung oder aus der AUS-Stellung jeweils in die Schaltstellung überführt werden, in welcher insbesondere ein Umschalten der Schalteinrichtung von der AUS-Stellung in die AN-Stellung oder umgekehrt möglich ist.

Besonders kompakt ausbilden lässt sich das chirurgische Kupplungssystem wenn das dritte Verriegelungselement an einem Halteglied angeordnet oder gelagert ist und wenn das Löseglied mit dem Halteglied direkt oder indirekt in Eingriff oder in Kontakt bringbar ist zum Überführen der Verriegelungseinrichtung aus der AN-Stellung oder aus der AUS-Stellung in die Schaltstellung. Durch einfaches Zusammenwirken des Löseglieds mit dem Halteglied kann so beispielsweise das dritte Verriegelungselement bewegt werden, um es mit dem ersten oder zweiten Verriegelungselement außer Eingriff zu bringen.

Der Aufbau des chirurgischen Kupplungssystems lässt sich auf einfache Weise insbesondere dadurch weiter vereinfachen, dass die Vorspanneinrichtung das Halteglied umfasst. Beispielsweise kann das Halteglied in Form eines Federelements, vorzugsweise eines Blattfederelements, ausgebildet sein, welches in Folge einer Auslenkung aus einer Grundstellung ohne Einwirken weiterer äußerer Kräfte wieder selbsttätig in die Grundstellung zurückgeht. Beispielsweise kann die Grundstellung eines solchen Halteglieds diejenige Stellung sein, in der das dritte Verriegelungselement mit einem der beiden anderen Verriegelungselemente in Eingriff steht.

Gemäß einer weiteren bevorzugten Ausführungsform der Erfindung kann vorgesehen sein, dass die Löseeinrichtung mindestens ein mechanisches Steuerelement umfasst, welches von einer Mitnahmestellung, in welcher das Löseglied mittels des Steuerelements direkt oder indirekt mit dem dritten Verriegelungselement in Kontakt oder Eingriff bringbar ist zum Überführen der Verriegelungseinrichtung aus der AN-Stellung oder aus der AUS-Stellung in die Schaltstellung, in eine Freigabestellung bringbar ist, in welcher das Löseglied weder direkt noch indirekt mit dem dritten Verriegelungselement hinreichend in Kontakt oder Eingriff bringbar ist zum Überführen der Verriegelungseinrichtung aus der AN-Stellung oder aus der AUS-Stellung in die Schaltstellung. Das Steuerelement ermöglicht es auf diese Weise, eine einfache mechanische Steuerung auszubilden, um mittels des Löseglieds die Verriegelungseinrichtung aus der AN- oder AUS-Stellung in die Schaltstellung zu überführen oder eben gerade nicht. Durch die spezielle Ausgestaltung des Steuerelements ist in der Freigabestellung ein Zusammenwirken zwischen dem Löseglied und dem dritten Verriegelungselement nicht möglich, um die Verriegelungseinrichtung von der AN- oder AUS-Stellung in die Schaltstellung zu überführen. Das Steuerelement kann so beispielsweise genutzt werden, um beim in Eingriff Bringen der Kupplungsvorrichtungen ein direktes Überfahren der AUS-Stellung zu vermeiden. Mit anderen Worten kann so eine Bewegung der Kupplungsvorrichtungen aufeinander zu bis in die AN-Stellung beispielsweise bei betätigtem Löseglied verhindert werden. Ebenso kann umgekehrt zum Beispiel beim Umschalten von der AN-Stellung in die AUS-Stellung ein unbeabsichtigtes Trennen der Kupplungsvorrichtungen voneinander verhindert werden.

Besonders kompakt lässt sich das Kupplungssystem ausbilden, wenn das Steuerelement in Richtung auf eine Längsachse des Kupplungssystems hin beweglich an der das dritte Verriegelungselement umfassenden Kupplungsvorrichtung angeordnet oder ausgebildet ist. So kann das Steuerelement räumlich sehr nahe zum dritten Verriegelungselement angeordnet beziehungsweise ausgebildet werden, was ein Zusammenwirken des Steuerelements und des dritten Verriegelungselements vereinfacht.

Günstig ist es, wenn die Löseeinrichtung derart ausgebildet ist, dass das Löseglied in der betätigten Stellung das Steuerelement von der Mitnahmestellung in die Freigabestellung überführt zum Verhindern, dass das Löseglied direkt oder indirekt mit dem dritten Verriegelungselement in Kontakt oder Eingriff bringbar ist zum Überführen der Verriegelungseinrichtung aus der AN-Stellung oder aus der AUS-Stellung in die Schaltstellung. Diese Ausgestaltung kann insbesondere verhindern, dass dann, wenn das Löseglied die betätigte Stellung einnimmt, es mit dem dritten Verriegelungselement in Kontakt oder in Eingriff gebracht werden kann, um die Verrieglungseinrichtung in die Schaltstellung zu überführen. Wie bereits dargelegt, kann so insbesondere ein unbeabsichtigtes Überfahren sowohl der AN-Stellung als auch der AUS-Stellung verhindert werden. Werden beispielsweise die beiden Kupplungsvorrichtungen miteinander in Eingriff gebracht und nehmen sie die Schaltstellung ein, so kann bei dauernd betätigtem Löseglied das Kupplungssystem nicht weiter in die AN-Stellung überführt werden. Um dies zu erreichen, muss erst das Löseglied in die unbetätigte Stellung überführt werden, so dass das Steuerelement wiederum von der Freigabestellung in die Mitnahmestellung übergehen kann, so dass eine erneute Betätigung des Löseglieds das außer Eingriff Bringen des dritten Verriegelungselements mit einem der beiden anderen Verriegelungselemente zur Folge hat. Es ist also erforderlich, dass eine Bedienperson das Löseglied, welches beispielsweise in radialer Richtung von der Längsachse weg weisend federnd vorgespannt angeordnet sein kann, zumindest kurz freigibt, um nach Erreichen der AUS-Stellung oder der AN-Stellung die Schalteinrichtung in die jeweils andere Stellung zu überführen oder die beiden Kupplungsvorrichtungen ganz voneinander zu trennen.

Vorteilhaft ist es, wenn die Löseeinrichtung derart ausgebildet ist, dass nur durch Überführen des Löseglieds von der betätigten Stellung in die unbetätigte Stellung das Steuerelement von der Freigabestellung in die Mitnahmestellung überführbar ist. Wie bereits dargelegt kann durch diese Ausgestaltung insbesondere sichergestellt werden, dass beim in Eingriff Bringen der Kupplungsvorrichtungen diese vorzugsweise zuerst in der AUS-Stellung automatisch verriegelt werden. Erst durch Überführen des Löseglieds von der betätigten Stellung in die unbetätigte Stellung wird das Steuerelement quasi wieder so aktiviert, dass in Folge einer weiteren Betätigung des Löseglieds das dritte Verriegelungselement wieder mit einem der beiden anderen Verriegelungselementen außer Eingriff gebracht werden kann. Beispielsweise kann das Kupplungssystem dann so ausgebildet sein, dass bei betätigtem Löseglied die Kupplungsvorrichtungen miteinander in Eingriff gebracht werden können und die Verriegelungseinrichtung das Kupplungssystem dann automatisch in der AUS-Stellung blockiert oder sichert. In einem nächsten Schritt kann nach Freigeben des Löseglieds das Steuerelement von der Freigabestellung in die Mitnahmestellung überführt werden. Nach nochmaligem Betätigen des Löseglieds kann dann das Kupplungssystem von der AUS-Stellung in die AN-Stellung überführt werden. In umgekehrter Reihenfolge lässt sich somit die Schalteinrichtung von der AN-Stellung in die AUS-Stellung und anschließend bei Bedarf die Schalteinrichtung wieder in die AN-Stellung oder das Kupplungssystem in die Trennstellung überführen. Mit anderen Worten kann bei betätigtem Löseglied das Kupplungssystem jeweils nur in die nächste Stellung gebracht werden, also beispielsweise von der Trennstellung in die AUS-Stellung oder von der AUS-Stellung in die AN-Stellung oder von der AN-Stellung in die AUS-Stellung oder von der AUS-Stellung in die Trennstellung. Das direkte Überführen des Kupplungssystems beispielsweise von der Trennstellung in die AN-Stellung oder von der AN-Stellung in die Trennstellung jeweils über die AUS-Stellung hinweg ist dadurch nicht möglich.

Vorteilhaft ist es, wenn die Löseeinrichtung derart ausgebildet ist, dass das Löseglied nur in der Mitnahmestellung direkt oder indirekt mit dem dritten Verriegelungselement in Kontakt oder Eingriff bringbar ist zum Überführen der Verriegelungseinrichtung aus der AN-Stellung oder aus der AUS-Stellung in die Schaltstellung. Dadurch kann insbesondere erreicht werden, dass das Löseglied insbesondere in der Freigabestellung nicht mit dem dritten Verriegelungselement in Kontakt oder Eingriff bringbar ist derart, dass die Verriegelungseinrichtung aus der AN-Stellung oder aus der AUS-Stellung in die Schaltstellung überführt werden kann.

Vorteilhaft ist es, wenn das Steuerelement mindestens einen ersten, dem ersten Verriegelungselement zugeordneten Steuervorsprung und mindestens einen zweiten, dem zweiten Verriegelungselement zugeordneten Steuervorsprung umfasst, welche quer zu einer Bewegungsrichtung des Löseglieds bewegbar sind zum Überführen des Steuerelements von der Mitnahmestellung in die Freigabestellung und umgekehrt. Beispielsweise dann, wenn eine Bewegungsrichtung des Löseglieds quer zu einer Längsachse des Kupplungssystems orientiert ist, um die Verriegelungseinrichtung von der AN- oder AUS-Stellung in die Schaltstellung zu überführen, kann eine Bewegung der Steuervorsprünge quer zur Bewegungsrichtung des Löseglieds, also insbesondere in Umfangsrichtung bezogen auf die Längsachse des Kupplungssystems oder senkrecht zur Längsachse erfolgen. Beispielsweise kann das Löseglied in der betätigten Stellung mit den Steuervorsprüngen derart in Kontakt kommen, dass diese quer zur angegebenen Bewegungsrichtung des Löseglieds beim Übergang von der betätigten Stellung in die unbetätigte Stellung von der Mitnahmestellung in die Freigabestellung überführt werden können. Dies ermöglicht es insbesondere, mit dem Löseglied in der betätigten Stellung bei einer Bewegung der Kupplungsvorrichtung aufeinander zu oder voneinander weg automatisch das Steuerelement von der Mitnahmestellung in die Freigabestellung zu überführen, um so zu verhindern, dass das Löseglied mit dem dritten Verriegelungselement direkt oder indirekt in Kontakt oder Eingriff bringbar ist, um die miteinander in Eingriff stehenden Verriegelungselemente außer Eingriff zu bringen.

Günstig ist es, wenn zwei erste und/oder zwei zweite Steuervorsprünge vorgesehen sind, welche in der Freigabestellung einen größeren Abstand voneinander aufweisen als in der Mitnahmestellung. Beispielsweise kann so eine Funktionssicherheit des Kupplungssystems erhöht werden. Insbesondere kann der Abstand zwischen den Steuervorsprüngen in der Freigabestellung so groß sein, dass das Löseglied zwischen diese ungehindert eingreifen kann und sie nicht beim Betätigen des Löseglieds oder Überführen desselben von der unbetätigten Stellung in die betätigte Stellung als Mitnehmer wirken können, um das dritte Verriegelungselement mit einem der beiden anderen Verriegelungselemente außer Eingriff zu bringen.

Vorteilhaft ist es, wenn die ersten Steuervorsprünge und/oder die zweiten Steuervorsprünge durch einen Schlitz voneinander getrennt sind, in welchen das Löseglied in der betätigten Stellung in Folge einer Bewegung der beiden Kupplungsvorrichtungen aufeinander zu oder voneinander weg eingreift zum zwangsweisen Bewegen der Steuervorsprünge in entgegengesetzte Richtungen voneinander weg in die Freigabestellung. Beispielsweise kann dies dadurch erfolgen, dass durch das Einführen des Löseglieds in der betätigten Stellung in den Schlitz die einander zugeordneten Steuervorsprünge voneinander weg verschwenkt oder verschoben werden, wodurch ein Abstand zwischen diesen vergrößert wird. Beispielsweise kann das Löseglied so kurz sein, dass es in der betätigten Stellung zwischen die in die Freigabestellung überführten Steuervorsprünge eingreift und dadurch weder direkt noch indirekt mit dem dritten Verriegelungselement in Kontakt treten kann, um dieses mit einem der beiden anderen Verriegelungselemente außer Eingriff zu bringen.

Günstig kann es ferner sein, wenn die Löseeinrichtung eine Rückstelleinrichtung umfasst zum automatischen Überführen des Steuerelements von der Freigabestellung in die Mitnahmestellung, sobald das Löseglied von der betätigten Stellung in die unbetätigte Stellung überführt wird. Um das durch das dauernd betätigte Löseglied quasi in seiner Funktion ausgeschaltete Steuerelement wieder in die Mitnahmestellung zu überführen, muss lediglich das Löseglied in die unbetätigte Stellung überführt werden. Die Rückstelleinrichtung überführt dann automatisch das Steuerelement von der Freigabestellung, in welcher es nicht als Mitnehmer für das Löseglied wirken kann, in die Mitnahmestellung.

Gemäß einer weiteren bevorzugten Ausführungsform der Erfindung kann vorgesehen sein, dass die erste und die zweite Kupplungsvorrichtung jeweils zwei erste und zwei zweite elektrische Kupplungskontakte umfassen. Beispielsweise können über die in der AUS-Stellung bereits miteinander in Kontakt stehenden zweiten elektrischen Kupplungskontakte durch Anlegen einer Messspannung und Ermitteln eines durch das Handstück fließenden Messstroms eine oder mehrere Kenngrößen desselben abgefragt werden, um die Art des Handstücks automatisch mit einer Steuer- und/oder Regelungsvorrichtung ermitteln und dann entsprechend eine Bestromung des Elektromotors des Handstücks vorgeben zu können.

Vorteilhaft ist es, wenn drei der vier elektrischen Kupplungskontakte jeweils mit einer Motorwicklung des Elektromotors elektrisch leitend in Verbindung stehen. So können beispielsweise drei Motorwicklungen des Elektromotors direkt über drei Kupplungskontakte angesteuert werden. Der vierte elektrische Kupplungskontakt kann beispielsweise indirekt über einen in Serie geschalteten Widerstand ebenfalls mit einer Motorwicklung elektrisch leitend in Verbindung stehen. Vorzugsweise handelt es sich beim vierten elektrischen Kupplungskontakt um einen der beiden zweiten elektrischen Kupplungskontakte.

Günstig kann es ferner sein, wenn das Kupplungssystem eine elektrische Versorgungsleitung mit einem ersten und einem zweiten Ende umfasst, wenn die erste Kupplungsvorrichtung am ersten Ende angeordnet oder ausgebildet ist und wenn das zweite Ende mit einer Steuer- und/oder Regelungsvorrichtung zum Steuern und/oder Regeln eines chirurgischen Elektromotors verbunden oder lösbar verbindbar ist. Selbstverständlich wäre es alternativ auch denkbar, die zweite Kupplungsvorrichtung am ersten Ende der Versorgungsleitung vorzusehen. Zum Beispiel kann die erste Kupplungseinrichtung der Versorgungsleitung mit einer zweiten Kupplungsvorrichtung gekuppelt oder in Eingriff gebracht werden, welche beispielsweise an einem chirurgischen Handstück, welches wahlweise mit oder ohne Elektromotor ausgestattet sein kann.

Vorteilhaft ist es, wenn das Kupplungssystem ein chirurgisches Handstück mit einem Elektromotor zum Antreiben eines chirurgischen Werkzeugs umfasst, welches mit dem Handstück koppelbar oder gekoppelt ist, welches Handstück ein proximales und ein distales Ende aufweist, an welchem proximalen Ende die zweite Kupplungsvorrichtung angeordnet oder ausgebildet ist. Ein solches chirurgisches Handstück kann dann beispielsweise mit einer eine erste Kupplungsvorrichtung umfassenden Versorgungsleitung gekoppelt oder in Eingriff gebracht werden. Sind die Versorgungsleitung und das Handstück miteinander mittels ihrer beiden Kupplungsvorrichtungen in Eingriff gebracht, kann das die beiden Kupplungsvorrichtungen umfassende Kupplungssystem wie oben beschrieben wahlweise auch zum Schalten, das heißt zum gezielten Betätigen des Elektromotors des Handstücks, genutzt werden. Weitere Schaltvorrichtungen zum An- beziehungsweise Ausschalten des Elektromotors sind dann nicht mehr zwingend erforderlich.

Um beispielsweise automatisch von einer Steuer- und/oder Regelungsvorrichtung ermitteln zu lassen, welche Art von Handstück mit dieser verbunden ist, ist es günstig, wenn das Handstück mindestens ein Kodierelement umfasst zum Kodieren der Art des Handstücks. Beispielsweise kann das Kodierelement in Form einer elektronischen Schaltung ausgebildet sein. Insbesondere kann es sich dabei um einen RFID-Chip handeln.

Auf besonders einfache Weise lässt sich eine Art des Handstücks kodieren, wenn das mindestens eine Kodierelement einen Widerumstand umfasst, welcher einen Widerstandswert aufweist, der der Art des Handstücks zugeordnet ist oder entspricht. Beispielsweise kann wie oben beschrieben in der AUS-Stellung durch Anlegen einer elektrischen Spannung an die zweiten elektrischen Kupplungskontakte und durch Messen des dann fließenden Stroms der Widerstandswert ermittelt werden, welcher einer bestimmten Art von Handstück entspricht. Bei Kenntnis der zu erwartenden Widerstandswerte verfügbarer Handstücke kann dann so die Art des konkret vorliegenden Handstücks auf einfache Weise ermittelt werden.

Vorzugsweise ist das mindestens eine Kodierelement elektrisch leitend mit einem zweiten Kupplungskontakt der zweiten Kupplungsvorrichtung verbunden. Auf diese Weise kann es beispielsweise in der AUS-Stellung benutzt werden, um die Art des Handstücks automatisch zu erkennen.

Gemäß einer weiteren bevorzugten Ausführungsform der Erfindung kann vorgesehen sein, dass das Handstück eine Werkzeugkupplungseinrichtung umfasst zum lösbaren Kuppeln des Handstücks mit einem Bearbeitungswerkzeug. Beispielsweise kann die Werkzeugkupplungseinrichtung derart angeordnet und ausgebildet sein, dass das Bearbeitungswerkzeug mit dem Handstück beziehungsweise mit dessen distalem Ende in Eingriff bringbar ist. Dies hat den Vorteil, dass mit demselben Handstück wahlweise unterschiedliche Bearbeitungswerkzeuge gekoppelt werden können, je nach durchzuführendem chirurgischem Eingriff.

Günstigerweise ist die Werkzeugkupplungseinrichtung von einer Werkzeugkupplungsstellung, in welcher das Handstück mit einem Bearbeitungswerkzeug gekoppelt ist, in eine Werkzeugtrennstellung, in welcher das Bearbeitungswerkzeug vom Handstück trennbar ist, bringbar. Eine derartige Werkzeugkupplungseinrichtung ermöglicht das einfache Lösen eines Bearbeitungswerkzeugs in der Werkzeugtrennstellung vom Handstück.

Besonders vorteilhaft ist, wenn die Werkzeugkupplungseinrichtung in der AN-Stellung blockiert ist zum Verhindern eines Überführens von der Werkzeugkupplungsstellung in die Werkzeugtrennstellung. Auf diese Weise kann insbesondere verhindert werden, dass eine Bedienperson versehentlich das Bearbeitungswerkzeug vom Handstück trennt, wenn das Kupplungssystem die AN-Stellung einnimmt. Mit anderen Worten kann so insbesondere verhindert werden, dass das Bearbeitungswerkzeug bei laufendem Elektromotor vom Handstück getrennt werden kann.

Günstig ist es, wenn die Werkzeugkupplungseinrichtung mindestens ein am Handstück bewegbar gehaltenes oder gelagertes Werkzeugkupplungsglied umfasst, welches nur in der Trennstellung oder in der AUS-Stellung bewegbar ist zum direkten oder indirekten Freigeben des Bearbeitungswerkzeugs, um es vom Handstück zu trennen, und wenn die Verriegelungseinrichtung in der AN-Stellung das mindestens eine Werkzeugkupplungsglied in der Werkzeugkupplungsstellung sichert. Beispielsweise kann so verhindert werden, dass eine Bedienperson das Werkzeugkupplungsglied betätigen oder bewegen kann, wenn das Kupplungssystem sich in der AN-Stellung befindet. So ist quasi die Werkzeugkupplungseinrichtung in der AN-Stellung gesichert oder gesperrt. Ein versehentliches Lösen des Bearbeitungswerkzeugs vom Handstück während eines Betriebs des Elektromotors ist dann nicht mehr möglich.

Auf besonders einfache Weise kann sichergestellt werden, dass die zweiten elektrischen Kupplungskontakte sowohl in der AN-Stellung als auch in der AUS-Stellung miteinander in Kontakt oder Eingriff stehen, wenn die ersten elektrischen Kupplungskontakte oder zumindest ein Teil derselben kürzer sind als die zweiten elektrischen Kupplungskontakte. Sie können beispielsweise beim aufeinander zu Bewegen der beiden Kupplungsvorrichtungen die zweiten elektrischen Kupplungskontakte der beiden Kupplungsvorrichtungen bereits miteinander in Kontakt treten oder in Eingriff gelangen, während dies für die ersten elektrischen Kupplungskontakte erst dann möglich ist, wenn die beiden Kupplungsvorrichtungen noch weiter aufeinander zu bewegt werden. So lassen sich auf einfache Weise die AN-Stellung und die AUS-Stellung durch entsprechende Relativ-Stellung der beiden Kupplungsvorrichtungen vorgeben.

Die eingangs gestellte Aufgabe wird ferner bei einem chirurgischen Antriebssystem der eingangs beschriebenen Art erfindungsgemäß dadurch gelöst, dass es eines der oben beschriebenen Kupplungssysteme zum elektrischen und mechanischen Verbinden des Handstücks und der Versorgungsleitung umfasst.

Ein derart weitergebildetes Antriebssystem ermöglicht somit auch das Schalten des Handstücks allein aufgrund der besonderen Ausbildung des Kupplungssystems. Eine weitere Schalteinrichtung ist dann nicht mehr zwingend erforderlich. Insgesamt weist das so verbesserte chirurgische Antriebssystem auch die oben im Zusammenhang mit bevorzugten Ausführungsformen chirurgischer Kupplungssysteme dargelegten Vorteile auf.

Zum Steuern und/oder Regeln des Elektromotors ist es vorteilhaft, wenn das Antriebssystem eine entsprechende Steuer- und/oder Regelungsvorrichtung umfasst. Diese kann insbesondere mit dem chirurgischen Handstück derart zusammenwirkend ausgebildet sein, dass sie die Art des Handstücks automatisch abfragen und erkennen kann, wenn das Kupplungssystem die AUS-Stellung einnimmt.

Die nachfolgende Beschreibung bevorzugter Ausführungsformen der Erfindung dient im Zusammenhang mit den Zeichnungen der näheren Erläuterung. Es zeigen:
- Figur 1:: eine schematische Gesamtdarstellung eines chirurgischen Antriebssystems;
- Figur 2:: eine schematische, perspektivische und teilweise durchbrochene Darstellung eines chirurgischen Kupplungssystems;
- Figur 3:: eine teilweise geschnittene perspektivische Darstellung einer ersten Kupplungsvorrichtung des Kupplungssystems mit unbetätigter Löseeinrichtung;
- Figur 3a:: eine ausschnittsweise Ansicht der Anordnung aus Figur 3 mit betätigter Löseeinrichtung;
- Figur 4:: eine perspektivische, teilweise geschnittene Ansicht einer zweiten Kupplungsvorrichtung des Kupplungssystems;
- Figur 5:: eine teilweise geschnittene Explosionsdarstellung der zweiten Kupplungsvorrichtung;
- Figur 5a:: eine perspektivische Ansicht des Verriegelungskörpers der zweiten Kupplungsvorrichtung;
- Figur 5b:: eine perspektivische Ansicht des Steuerelements der zweiten Kupplungsvorrichtung;
- Figur 6:: eine teilweise durchbrochene Längsschnittansicht des Kupplungssystems vor dem in Eingriff Bringen elektrischer Kupplungskontakte der beiden Kupplungsvorrichtungen;
- Figur 7a:: eine Ansicht des Kupplungssystems analog Figur 6 in der AUS-Stellung;
- Figur 7b:: eine teilweise geschnittene Ansicht in Richtung des Pfeils A in Figur 7a;
- Figur 8a:: eine Ansicht des Kupplungssystems analog Figur 7a mit unbetätigtem Löseglied;
- Figur 8b:: eine Ansicht analog Figur 7b der Anordnung des Kupplungssystems aus Figur 7a in Richtung des Pfeils B;
- Figur 9:: eine Ansicht des Kupplungssystems analog Figur 8a mit betätigtem Löseglied in der Mitnahmestellung des Steuerelements;
- Figur 10a:: eine Ansicht des Kupplungssystems analog Figur 9 in der Schaltstellung beim Übergang von der AUS-Stellung in die AN-Stellung;
- Figur 10b:: eine Ansicht analog Figur 8b der Anordnung des Kupplungssystems aus Figur 10a in Richtung des Pfeils C;
- Figur 11a:: eine Ansicht des Kupplungssystems analog Figur 10a in der AN-Stellung;
- Figur 11b:: eine Ansicht analog Figur 10b der Anordnung des Kupplungssystems in Figur 11a in Richtung des Pfeils D;
- Figur 12a:: eine Ansicht des Kupplungssystems analog Figur 11a mit unbetätigtem Löseglied in der Kupplungsstellung;
- Figur 12b:: eine Ansicht analog Figur 11b der Anordnung des Kupplungssystems aus Figur 12a in Richtung des Pfeils E;
- Figur 13:: eine Ansicht des Kupplungssystems analog Figur 12a mit betätigtem Löseglied und dem Steuerelement in der Mitnahmestellung;
- Figur 14:: eine ausschnittsweise perspektivische Darstellung eines zweiten Ausführungsbeispiels eines chirurgischen Kupplungssystems in der Trennstellung;
- Figur 15:: eine ausschnittsweise Längsschnittansicht des Kupplungssystems aus Figur 14 in der AUS-Stellung;
- Figur 16a:: eine schematische Schaltskizze des Kupplungssystems in der Trennstellung;
- Figur 16b:: eine schematische Schaltskizze des Kupplungssystems in der AUS-Stellung; und
- Figur 16c:: eine schematische Schaltskizze des Kupplungssystems in der AN-Stellung.

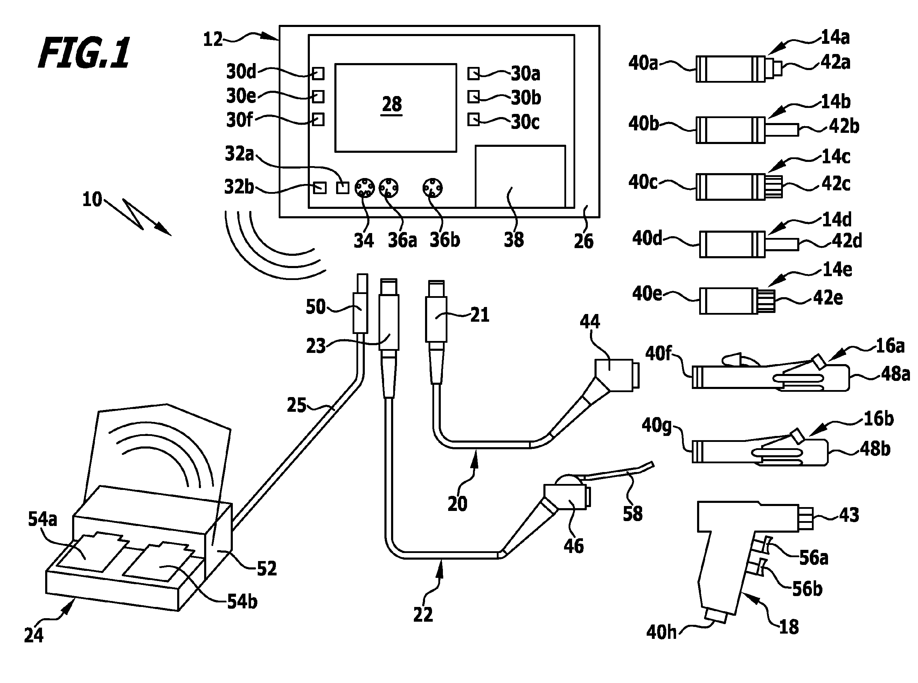

In Figur 1 ist schematisch ein insgesamt mit dem Bezugszeichen 10 versehenes chirurgisches Antriebssystem dargestellt, umfassend eine Steuer- und/oder Regelungseinrichtung in Form eines Steuergeräts 12, fünf Handstücke 14a bis 14e, zwei Shaverhandstücke 16a und 16b, ein Pistolenhandstück 18, zwei Versorgungsleitungen in Form von Anschlusskabeln 20 und 22 sowie eine Fußsteuerung 24. Alle genannten Handstücke umfassen einen integrierten Elektromotor als Antrieb und bilden so Antriebseinheiten.

Das Steuergerät 12 umfasst einen in einem Gehäuse 26 angeordneten flachen Bildschirm 28 in Form eines Touchscreens. Zu beiden Seiten des Bildschirms 28 sind je drei Bedienelemente 30a bis 30c beziehungsweise 30d bis 30f angeordnet.

Zwei Schalter 32a und 32b sind unterhalb des Bildschirms 28 auf einer Linie angeordnet mit einer Anschlussbuchse 34 zum Anschluss der Fußsteuerung 24 über ein optionales Anschlusskabel 25 und mit zwei Anschlussbuchsen 36a und 36b zum Anschluss der Anschlusskabel 20 und 22, mit denen die Handstücke mit dem Steuergerät 12 verbunden werden können. Optional kann außerdem ein Anschluss 38 für ein Fluidsystem zur Zufuhr und Abfuhr von Fluiden aus einem Operationsbereich vorgesehen sein, beispielsweise auch zur Versorgung von Spül- oder Absaugkanälen an mit den Handstücken 14, den Shaverhandstücken 16 oder dem Pistolenhandstück 18 verbindbaren, nicht dargestellten Getriebeeinheiten oder Werkzeugen, mit welchen zusammen die Handstücke chirurgische Instrumente des Antriebssystems 10 bilden.

Die Handstücke 14a bis 14e umfassen jeweils eine Kabelkupplung 40a bis 40e, die mit einem Kupplungsstück 44 des Anschlusskabels 20 oder einem Kupplungsstück 46 des Anschlusskabels 22 beliebig verbindbar sind. Ebenso weisen die beiden Shaverhandstücke 16a und 16b sowie das Pistolenhandstück 18 jeweils eine Kabelkupplung 40f, 40g beziehungsweise 40h auf, die mit einem der beiden Kupplungsstücke 44 oder 46 verbindbar sind.

An ihrem jeweils anderen Ende sind die Handstücke 14a bis 14e mit Getriebeoder Werkzeugkupplungen ausgestattet, welche Werkzeugkupplungseinrichtungen 42a bis 42e definieren, auf die nicht dargestellte Getriebeeinheiten, beispielsweise ausgestattet mit Bohrern, Sägeblättern oder dergleichen angekuppelt und durch die Handstücke 14a bis 14e angetrieben werden können. Je nach Ausgestaltung können die Handstücke 14a bis 14e auch direkt mit nicht dargestellten Bearbeitungswerkzeugen, wie beispielsweise Bohrern oder Sägeblättern, bestückt werden zur Ausbildung chirurgischer Instrumente.

Die Handstücke 14a bis 14e sind vorzugsweise sensorlos ausgebildet, das heißt sie weisen keine Sensoren auf, um eine Drehzahl der Handstücke 14a bis 14e während des Betriebs zu bestimmen. Die Handstücke des Antriebssystems 10 unterscheiden sich nicht nur, wie in Figur 1 schematisch dargestellt, äußerlich, sondern auch hinsichtlich ihres inneren Aufbaus. Dies bedeutet, dass die in den Handstücke 14a bis 14e verbauten Elektromotoren unterschiedlichen Typs sein und sich beispielsweise in ihren Kenngrößen, wie zum Beispiel Minimaldrehzahl, Maximaldrehzahl, Maximalstrom und Maximaldrehmoment, unterscheiden können. Zudem können, wie bei den beiden Shaverhandstücken 16a und 16b, Getriebe integriert sein, welche optional auch in an die Handstücke 14a bis 14e sowie an das Pistolenhandstück 18 ankoppelbare Getriebeeinheiten integriert sein können. Die Getriebeeinheiten können je nach Ausgestaltung auch selbst zusätzlich mit unterschiedlichen Instrumentenspitzen in Form chirurgischer Werkzeuge bestückt werden.

Des Weiteren umfassen die Shaverhandstücke 16a und 16b jeweils eine Shaverkupplung 48a beziehungsweise 48b zum Anschluss eines Shaveraufsatzes, beispielsweise zur Anwendung in der Arthroskopie.

Die Anschlusskabel 20 und 22 sind zum Verbinden mit dem Steuergerät mit Kupplungen 21 und 23 versehen, über die sie mit den Anschlussbuchsen 36a und 36b verbindbar sind.

Die Fußsteuerung 24 steht über eine drahtlose Datenübertragungseinrichtung mit dem Steuergerät 12 in Verbindung, beispielsweise über ein Infrarot- oder Funkübertragungssystem. Optional ist auch eine Verbindung der Fußsteuerung 24 über ein mit der Anschlussbuchse 34 verbindbares Kupplungsstück 50 des Anschlusskabels 25 möglich. An einem Gehäuse 52 der Fußsteuerung 24 sind zwei fußbetätigbare Schalter 54a und 54b angeordnet, über die insbesondere ein Links- beziehungsweise Rechtslauf der Handstücke geregelt werden kann.

Das Pistolenhandstück 18 ist mit zwei Gebern 56 ausgestattet, wobei der Geber 56a beispielsweise zur Aktivierung eines Motorrechtslaufes, der Geber 56b zur Aktivierung eines Motorlinkslaufes vorgesehen sein können.

Die Anschlusskabel 20 und 22 unterscheiden sich dadurch, dass am Anschlusskabel 22, anders als am Anschlusskabel 20, ein Betätigungshebel 58 vorgesehen ist, mit dem eine Bedienperson einen Motorbetrieb eines Handstücks 14, eines Shaverhandstücks 16 oder des Pistolenhandstücks 18 aktivieren kann. Der Betätigungshebel 58 hat die Funktion eines Drehzahlgebers, mit dem eine Drehzahl des Motors von einer Bedienperson vorgegeben werden kann.

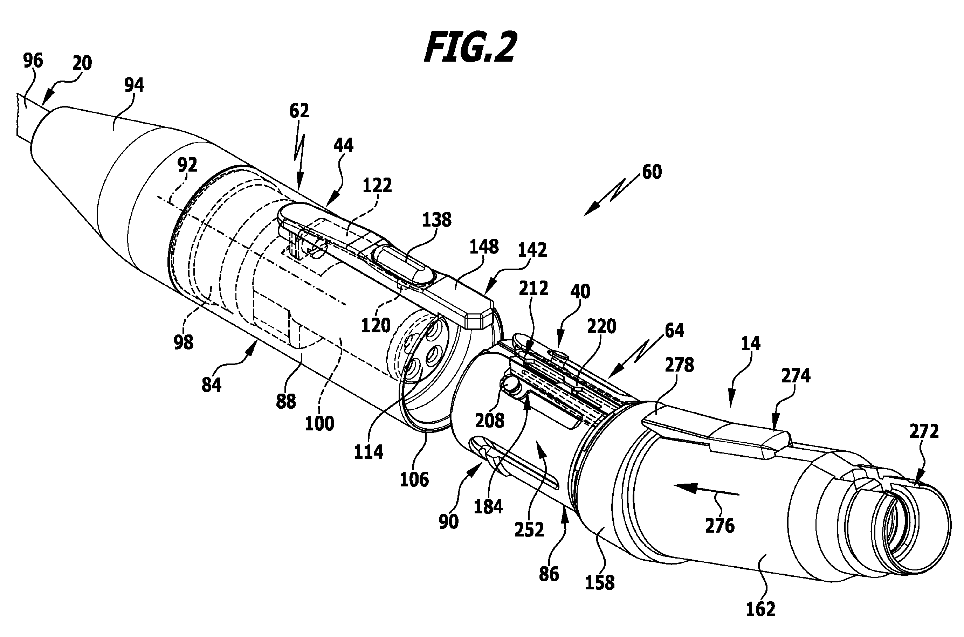

Zusätzlich zu dem beim Anschlusskabel 22 vorgesehenen Betätigungshebel 58, welcher somit eine am Anschlusskabel 22 ausgebildete Drehzahlvorgabeeinrichtung bildet, kann auch ein in Figur 2 schematisch dargestelltes und insgesamt mit dem Bezugszeichen 60 bezeichneten chirurgisches Kupplungssystem vorgesehen sein. So können sowohl das Kupplungsstück 44 am Anschlusskabel 20 als auch das Kupplungsstück 46 am Anschlusskabel 22 in Form einer ersten Kupplungseinrichtung 62 ausgebildet sein, eine Kabelkupplung 40 an dem in Figur 2 schematisch dargestellten Handstück 14 in Form einer zweiten Kupplungseinrichtung 64. Das Kupplungssystem 60 ist in Figur 2 in einer Trennstellung dargestellt, in welcher die beiden Kupplungsvorrichtungen 60 und 62 vollständig voneinander getrennt sind, also insbesondere mechanisch außer Eingriff stehen. Jede der beiden Kupplungsvorrichtungen 62 und 64 umfasst vier elektrische Kupplungskontakte. Die erste Kupplungsvorrichtungen 62 umfasst die Kupplungskontakte 66, 67, 68 und 69, die zweite Kupplungsvorrichtungen 64 umfasst die Kupplungskontakte 70, 71, 72 und 73. Das Anschlusskabel 20 umfasst lediglich drei Leitungen 74, 75 und 76, über welche drei Motorwicklungen 78, 79 und 80 eines in das Handstück 14 integrierten Elektromotors 82 mit Strom versorgt werden können. Dabei ist der Kupplungskontakt 66 elektrisch leitend mit der Leitung 74 verbunden, der Kupplungskontakt 67 mit der Leitung 75. Des Weiteren sind die beiden Kupplungskontakte 68 und 69 elektrisch leitend mit der Leitung 76 verbunden.

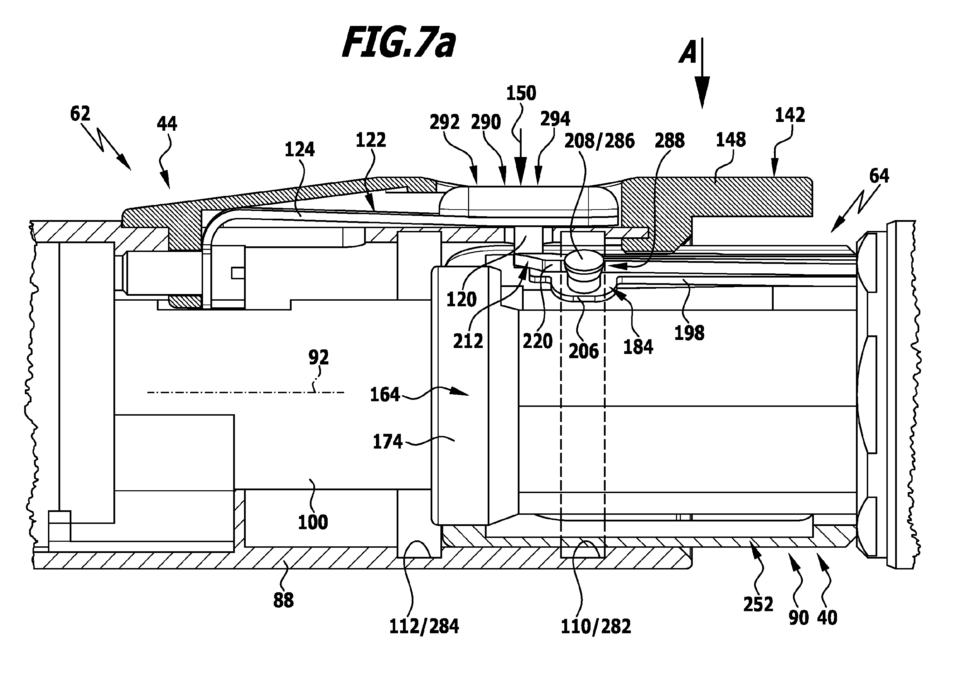

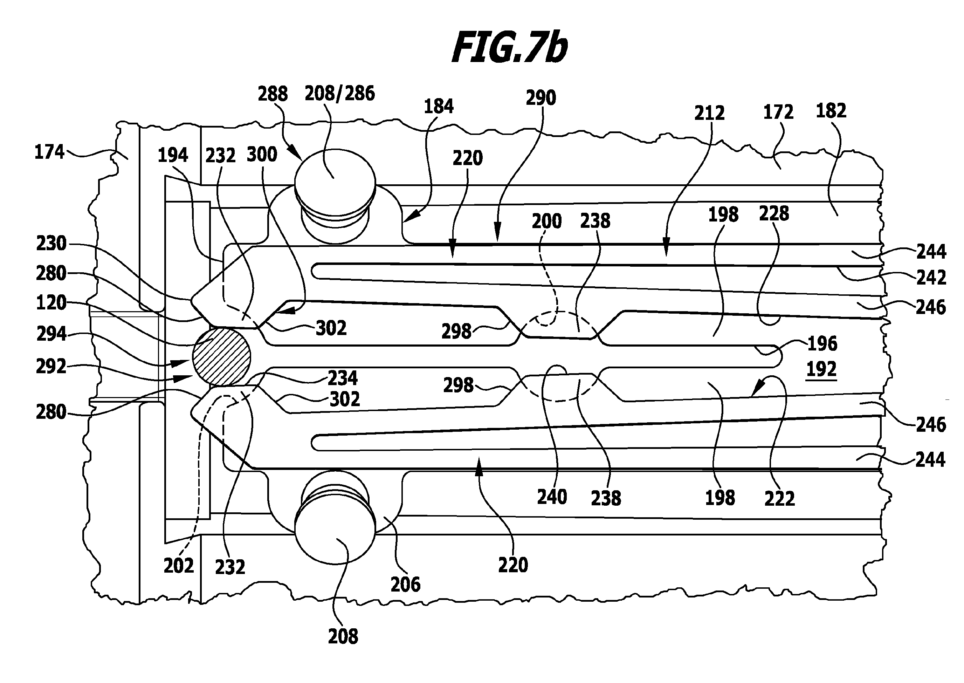

Zum mechanischen Kuppeln der beiden Kupplungsvorrichtungen 62 und 64 umfasst die erste Kupplungsvorrichtung 62 ein erstes Kupplungselement 84 und die zweite Kupplungsvorrichtung 64 ein zweites Kupplungselement 86. Diese können in einer Kupplungsstellung miteinander in Eingriff gebracht werden. In Figur 7 ist das Kupplungssystem 60 schematisch in der Kupplungsstellung dargestellt. Das erste Kupplungselement 84 ist vorzugsweise in Form einer Kupplungshülse 88 ausgebildet, in welche das in Form eines Kupplungszapfens 90 ausgebildet zweite Kupplungselement 86 in einer Richtung parallel zu einer Längsachse 92 des Kupplungssystems 60 eingeführt werden kann.

Die erste Kupplungsvorrichtung 62 umfasst ein proximales Ende, welches in Form einer als Zugentlastung dienenden Kabelschutzhülle 94 ausgebildet ist, aus welcher ein drei Leitungen 74, 75 und 76 umfassendes Kabel 96 herausgeführt ist. In distaler Richtung erstreckt sich von der Kabelschutzhülle 94 ein im Wesentlichen rotationssymmetrischer Lagerkörper 98, welcher von der in distaler Richtung weisenden Kupplungshülse 88 umgeben ist. Innerhalb der Kupplungshülse 88 erstreckt sich ausgehen vom Lagerkörper 98 ein zylindrischer Buchsenkörper 100, welcher vier im Inneren aus elektrisch leitfähigem Material ausgebildete Steckbuchsen 102, 103, 104 und 105 umfasst. Die Steckbuchsen 102, 103, 104 und 105 bilden die elektrischen Kupplungskontakte 66, 67, 68 und 69. Sie sind in distaler Richtung weisend geöffnet und mit ihren Längsachsen parallel zur Längsachse 92 ausgerichtet. Etwas beabstandet von einem distalen Ende 106 der Kupplungshülse 88 sind in einer Innenwand 108 derselben zwei umlaufende Ringnute 110 und 112 angeordnet, welche Verriegelungsausnehmungen definieren und in axialer Richtung voneinander beabstandet sind. Die Ringnut 110 weist vom Ende 106 in etwa denselben Abstand auf wie ein distales Ende 114 des Buchsenkörpers 100.

Etwas proximalseitig der Ringnut 110 ist die Kupplungshülse 88 mit einer quer zur Längsachse 92 orientierten Bohrung 116 versehen, in welche ein kurzer zylindrischer Stift 118 hineinragt, welcher ein Löseglied 120 bildet und an einem Lösehebel 122 senkrecht abstehend angeordnet ist. Der Lösehebel 122 ist aus einer L-förmigen Blattfeder 124 gebildet, welche einen kurzen Schenkel 126 umfasst, welcher mit einer Schraube 128, deren Längsachse sich parallel zur Längsachse 92 erstreckt, am Lagerkörper 98 gehalten ist. Ein langer Schenkel 130 der Blattfeder 124 ragt durch eine Durchbrechung 132 der Kupplungshülse 88 heraus und erstreckt sich im Wesentlichen parallel zur Längsachse 92 außerhalb der Kupplungshülse 88 in distaler Richtung. Ausgehend von einem distalen Ende 134 des langen Schenkels 130 ist auf einer Außenseite 136 desselben ein Betätigungsvorsprung 138 angeordnet. Dieser ragt durch eine langlochartige Durchbrechung 140 eines im Wesentlichen langgestreckt quaderförmigen Führungskörpers 142 heraus. Der Führungskörper 142 weist einen in Richtung auf die Längsachse 92 hin weisenden Vorsprung 144 auf, welcher proximalseitig in die Durchbrechung 132 eingreift und einerseits an einem Rand derselben und andererseits am Schenkel 126 anliegt. Er ist ebenfalls mit der Schraube 128 am Lagerkörper 98 gesichert. Distalseitig ist am Führungskörper 142 eine in proximaler Richtung weisende Aussparung 146 ausgebildet, in welche das Ende 106 eingreift. Zur eindeutigen Positionierung des Führungsköpers 142 an der Kupplungshülse 88 ist diese ausgehend vom Ende 106 in Umfangsrichtung entsprechend einer Breite des Führungskörpers 142 etwas dünner ausgebildet, so dass eine eindeutige Positionierung des Führungskörpers in Umfangsrichtung vorgegeben wird. Ein distales Ende des Führungsköpers 142 steht in distaler Richtung weisend parallel zur Längsachse 92 über das Ende 106 vor und bildet ein Blockierglied 148, dessen Funktion weiter unten näher beschrieben wird.

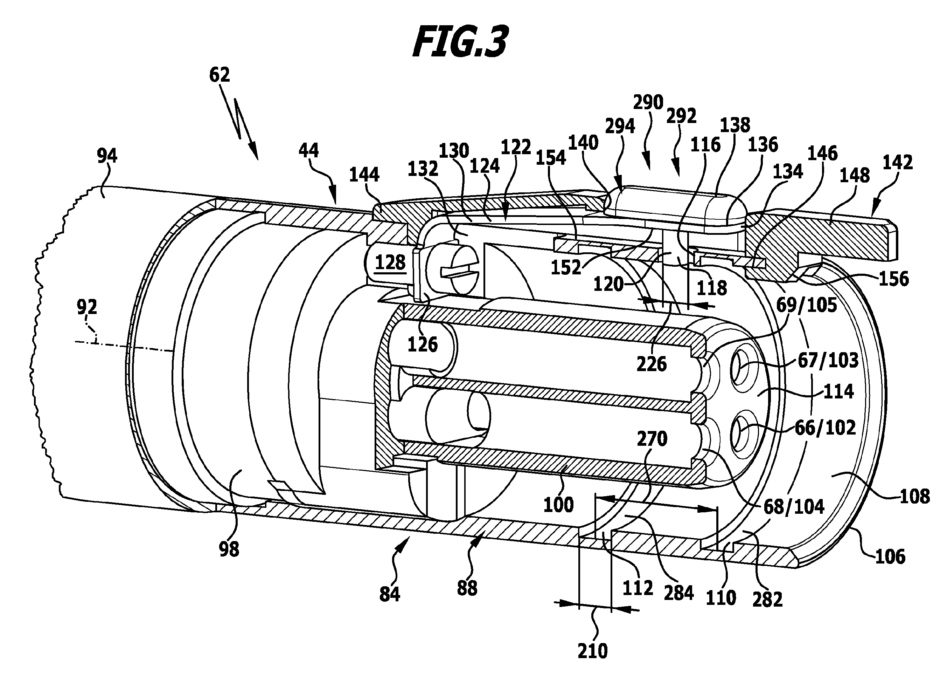

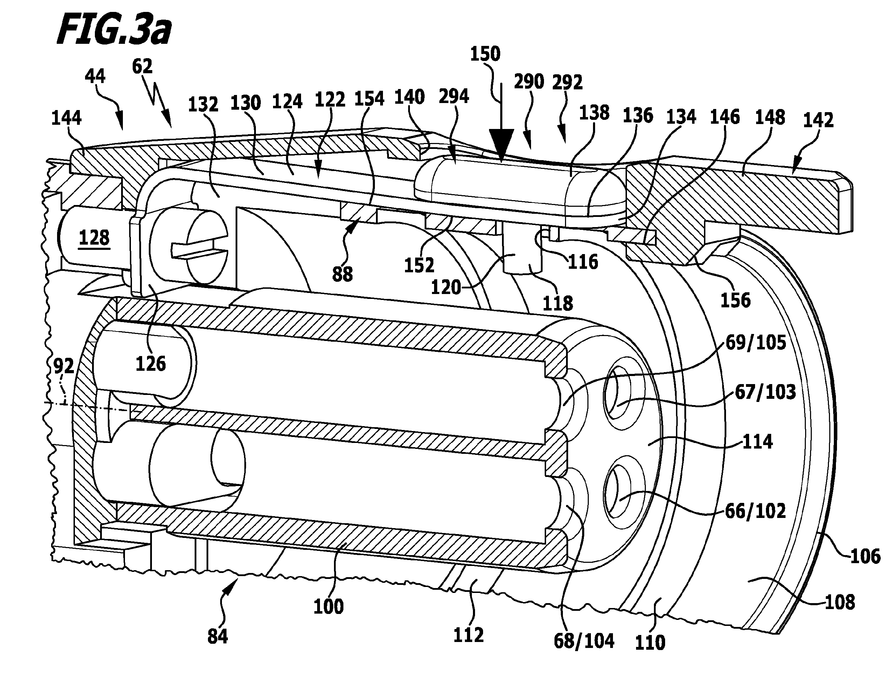

Wie in Figur 3a schematisch dargestellt, kann durch Kraftbeaufschlagung des Betätigungsvorsprungs 138 in Richtung des Pfeils 150, also quer zur Längsachse 92 und auf diese hin gerichtet, der Lösehebel 122 mit seinem in distaler Richtung weisenden Ende in Richtung auf die Längsachse 92 hin verschwenkt werden, bis eine Unterseite 152 des Schenkels 130 an einer Außenseite 154 der Kupplungshülse 88 anschlägt. In Figur 3a ist somit die betätigte Stellung des Löseglieds 120 dargestellt, in Figur 2 dessen unbetätigte Stellung. Der Führungskörper 142 umfasst ferner eine Führungsnase 156, welche von der Innenwand 108 etwas in Richtung auf die Längsachse 92 hin vorsteht. Sie dient der Zentrierung beim in Eingriff Bringen der beiden Kupplungsvorrichtungen 62 und 64.

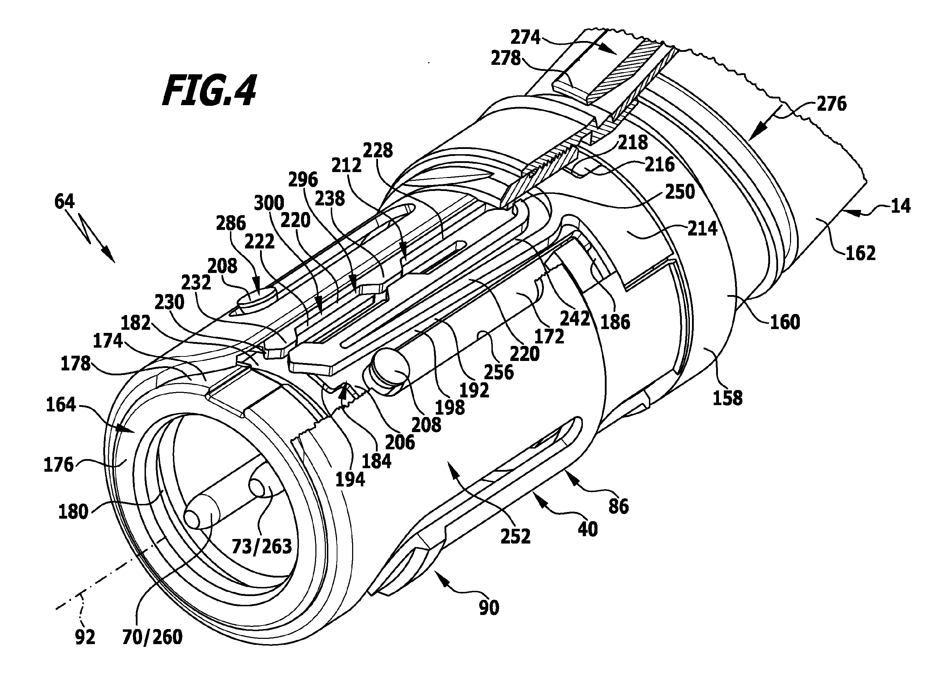

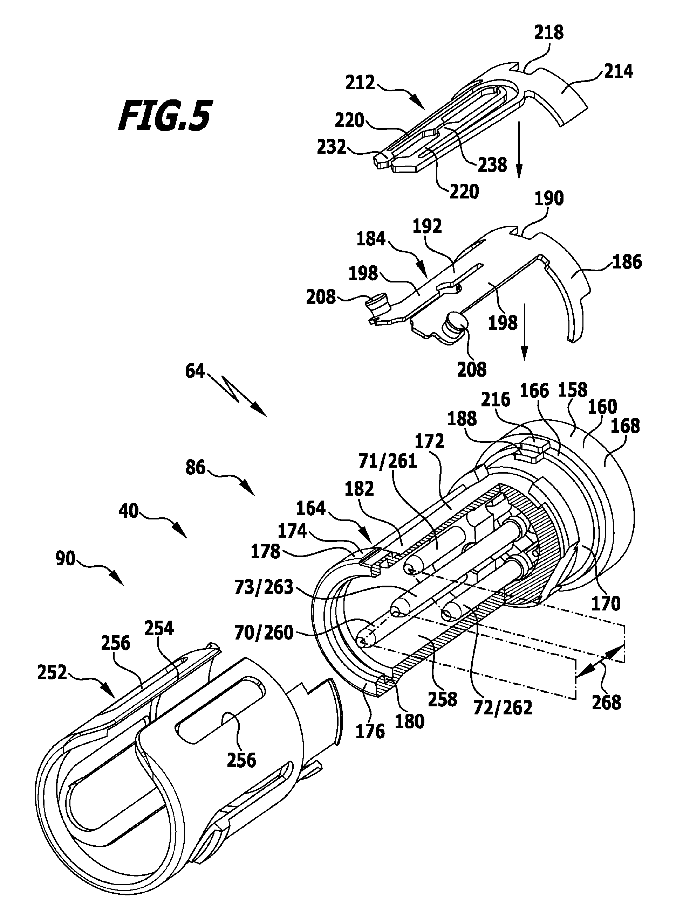

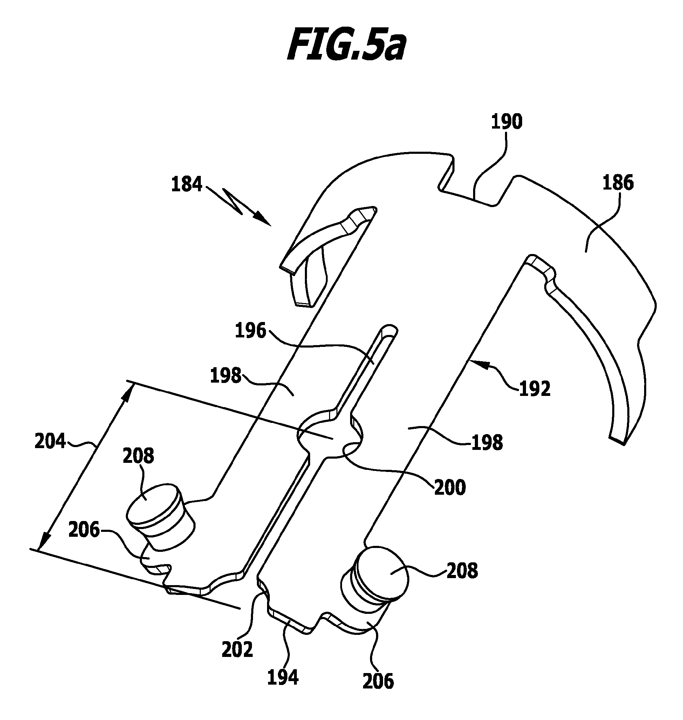

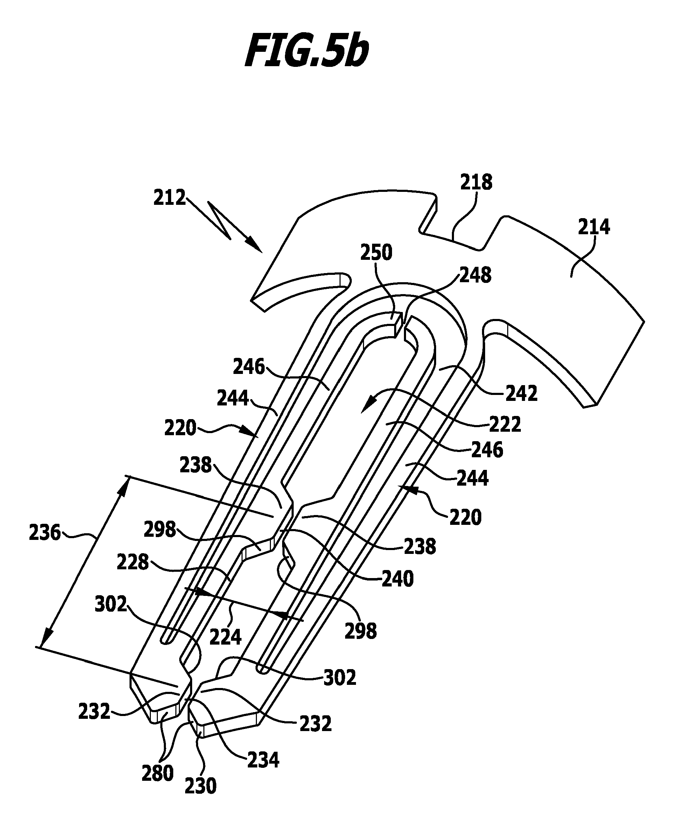

Der Aufbau der zweiten Kupplungsvorrichtung 62 wird nachfolgend in Verbindung mit den Figuren 4 und 5 näher erläutert.

Ein rotationssymmetrischer Lagerkörper 158 definiert gleichzeitig ein distales Ende 160 der zweiten Kupplungsvorrichtung 64, an welches sich ein Gehäuse 162 des Handstücks 14 anschließt. Der Kupplungszapfen 90 steht somit in proximaler Richtung weisend vom Lagerkörper 158 und damit vom Gehäuse ab. Er ist nicht massiv, sondern mehrteilig ausgebildet. Einstückig mit dem Lagerkörper 158 ausgebildet ist eine Zapfenhülse 164. Diese umfasst einen kurzen Abschnitt 166, welcher im Außendurchmesser gegenüber einem zylindrischen Innenabschnitt 168 etwas verringert ist. Proximalseitig schließt sich an den Abschnitt 166 ein weiterer zylindrischer Abschnitt 170 an, dessen Außendurchmesser gegenüber dem des Abschnitts 166 nochmals etwas verringert ist. Proximalseitig schließt sich an den Abschnitt 170 ein weiterer zylindrischer Abschnitt 172 an, welcher insgesamt etwas mehr als die Hälfte einer Gesamtlänge der Zapfenhülse 164 ausmacht. Ein letzter im Wesentlichen zylindrischer Abschnitt 174 bildet einen sich bis zum proximalen Ende 176 erstreckenden Ringflansch 178. Dieser ist im Inneren mit einer Ringnut 180 versehen.

Auf einer Außenseite ist der Abschnitt 172 mit einer flachen Aussparung 182 versehen, in welche ein Verriegelungskörper 184 eingesetzt ist. Dieser ist aus einem elastisch federnden Material hergestellt und umfasst einen ein distales Ende definierenden Halteabschnitt 186, welcher sich über einen Umfangswinkel von 180° erstreckt. Eine innere Krümmung des Halteabschnitts 186 ist an den Abschnitt 170 angepasst. Zur eindeutigen Positionierung des Halteabschnitts 186 an der Zapfenhülse 164 ist auf dem Abschnitt 170 direkt an den Abschnitt 166 angrenzend eine quaderförmige Nase 188 ausgebildet, welche in eine korrespondierende, in distaler Richtung weisende Aussparung 190 am Halteabschnitt 186 eingreift.

Vom Halteabschnitt 186 erstreckt sich in proximaler Richtung ein Halteglied 192, welches ausgehend von einem proximalen Ende 194 mit einem Schlitz 196 versehen ist. So werden praktisch zwei federelastische Haltearme 198 ausgebildet, welche symmetrisch zu einer die Längsachse 92 enthaltenden Spiegelebene 92 ausgebildet sind. Das Halteglied 192 ist ferner mit zwei quer zur Längsachse 92 orientierten Durchbrechungen 200 und 202 versehen. Die Durchbrechung 202 ist direkt ausgehend vom Ende 194 ausgebildet und im Wesentlichen halbkreisförmig. Die Durchbrechung 200 weist von der Durchbrechung 202 einen Abstand 204 auf.

Die Haltearme 198 sind jeweils in Umfangsrichtung mit voneinander weg weisenden Haltelappen 206 ausgestattet, die jeweils einen in radialer Richtung von der Längsachse 92 weg weisenden, rotationssymmetrischen Verriegelungsvorsprung 208 tragen. Ein Durchmesser der Verriegelungsvorsprünge 208 entspricht etwa einer Breite 210 der Ringnuten 110 und 112. Die Aussparung 182 ist so ausgebildet und bemessen, dass die Haltearme 198 bei entsprechender Kraftbeaufschlagung etwas in Richtung auf die Längsachse 92 hin verschwenkbar sind. Die Verriegelungsvorsprünge 208 bilden somit in radialer Richtung bewegbare Zapfen.

Auf einer von der Längsachse 92 weg weisenden Außenseite des Halteglieds 192 liegt teilweise ein Steuerelement 212 an. Es umfasst einen Halteabschnitt 214, welcher den Halteabschnitt 186 teilweise überdeckt. Zur eindeutigen Positionierung des Steuerelements 212 relativ zum Lagerkörper 158 ist am Abschnitt 166 in radialer Richtung vorstehend eine schmale quaderförmige Nase 216 ausgebildet, die in eine korrespondierende Aussparung 218 am Halteabschnitt 214 eingreift. Das Steuerelement 212 ist wie das Halteglied 192 spiegelsymmetrisch zu einer die Längsachse 92 enthaltenden Spiegelebene ausgebildet. Es weist zwei vom Halteabschnitt 214 in proximaler Richtung und im Wesentlichen parallel zur Längsachse 92 abstehende Steuerarme 220 auf. Diese sind durch einen Spalt 222 voneinander getrennt. Eine Breite 224 des Spalts 222 in Umfangsrichtung ist etwas größer als ein Durchmesser 226 des Löseglieds 120. Von aufeinander zu weisenden Innenkanten 228 der Steuerarme 220 stehen aufeinander zu weisend am proximalen Ende 230 der Steuerarme 220 zwei Steuervorsprünge 232 ab, die durch einen Spalt 234 voneinander getrennt sind. In einem Abstand 236 hiervon in distaler Richtung stehen von den Innenkanten 228 zwei zweite Steuervorsprünge 238 aufeinander zu weisend ab, die durch einen weiteren Spalt 240 voneinander getrennt sind. Des Weiteren weist das Steuerelement 212 einen U-förmigen Schlitz 242 auf, welcher jeden Steuerarm 220 in zwei im Wesentlichen parallel zueinander verlaufende Steuerschenkel 244 und 246 trennt. Distale Enden der die Steuervorsprünge 232 und 238 tragenden Steuerschenkel 244 sind durch einen weiteren Spalt 248 voneinander getrennt.

Die besondere Ausgestaltung des Steuerelements 212 ermöglicht es, dass die Steuerarme 220 mit ihren Enden 230 in Richtung auf die Längsachse 92 hin und wieder zurück verschwenkt werden können. Des Weiteren können die Enden 230 in Umfangsrichtung voneinander weg und wieder zurück verschwenkt werden, um die Spalte 234 und 240 zu vergrößern. Des Weiteren können distale Enden 250 der Steuerschenkel 246 in Umfangsrichtung voneinander weg und wieder zurück verschwenkt werden, um den Spalt 248 zu vergrößern.

Den Lagerkörper 158 umgibt ausgehend vom Ende 176 eine sich in distaler Richtung erstreckende Schutzhülse 252. Diese weist einen durchgehenden Längsschlitz 254 auf, zu dem parallel ferner zwei Langlöcher 256 angeordnet sind. Die Langlöcher 256 sind in ihrer Breite so bemessen, dass die Verriegelungsvorsprünge 208 durch sie hindurch greifen können. Eine äußere Kontur des Kupplungszapfens 90 wird daher im Wesentlichen bestimmt durch eine von der Schutzhülse 252 definierte Einhüllende, über die in einer Grundstellung die Verriegelungsvorsprünge 208 in radialer Richtung etwas vorragen.

Der Lagerkörper 158 ist ausgehend von seinem Ende 176 mit einem Sackloch 258 versehen. Im Sackloch 258 sind vier Kontaktstifte 260, 261, 262 und 263 angeordnet, welche freie, in proximaler Richtung weisende Ende aufweisen und korrespondierend zu den Steckbuchsen 102, 103, 104 und 105 ausgebildet sind. Die Kontaktstifte 260, 261, 262 und 263 bilden die Kupplungskontakte 70, 71, 72 und 73.

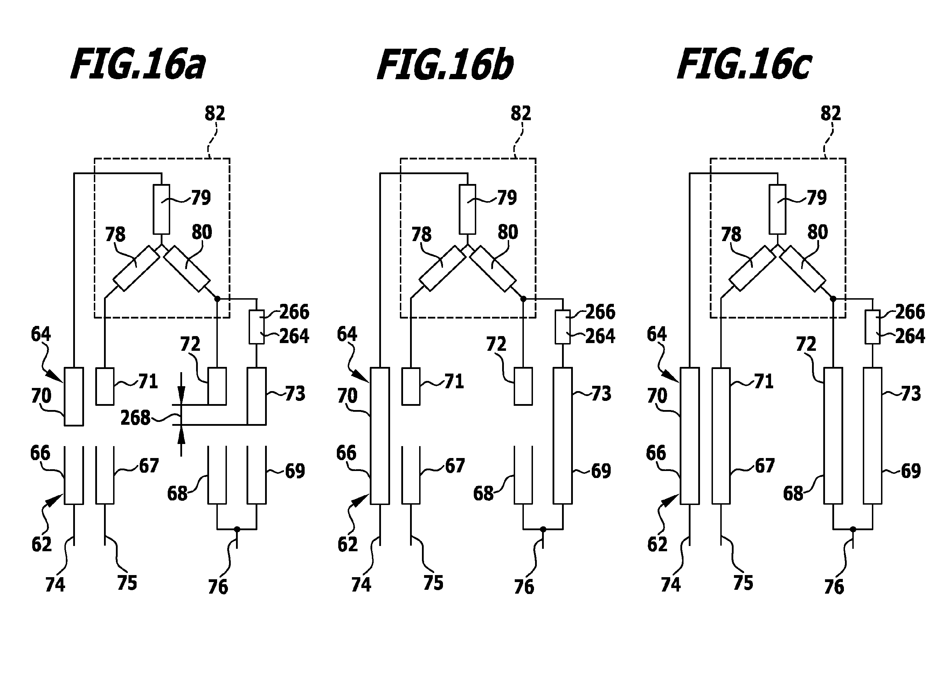

Der Kupplungskontakt 70 ist, wie in Figur 16a schematisch dargestellt, mit der Motorwicklung 79 elektrisch leitend verbunden. Die Motorwicklung 79 ist sternförmig mit den Motorwicklungen 78 und 80 verschaltet. Die Motorwicklung 78 ist ferner elektrisch leitend mit dem Kupplungskontakt 71 verbunden. Die Motorwicklung 80 ist einerseits elektrisch leitend mit dem Kupplungskontakt 72 verbunden und andererseits mit einem Widerstand 264, welcher ein Kodierelement 266 bildet zum Kodieren der Art des Handstücks 14. Ein Widerstandswert des Widerstands 264 ist eindeutig einer Art beziehungsweise einem Typ des Handstücks 14 zugeordnet beziehungsweise entspricht dieser beziehungsweise diesem. Insbesondere kann die Zuordnung bestimmt sein durch die Art des Elektromotors 82, so dass eine an das Handstück 14 angepasste Ansteuerung des Elektromotors 82 mit dem Steuergerät 12 erfolgen kann. Der Widerstand 264 ist in Serie zwischen die Motorwicklung 80 und den Kupplungskontakt 73 geschaltet.

Die Kontaktstifte 261 und 262 sind kürzer als die Kontaktstifte 260 und 263. Ein Abstand 268 zwischen freien Enden der Kontaktstifte 261 und 262 einerseits und 260 und 263 andererseits parallel zur Längsachse 92 ist etwas größer als der Abstand 270 der Ringnuten 110 und 112 voneinander.

Am Gehäuse 162 ist ferner eine Werkzeugkupplungseinrichtung 272 vorgesehen zum lösbaren Kuppeln eines distalen Endes des Handstücks 14 mit einem nicht näher dargestellten Bearbeitungswerkzeug. Sie umfasst ein Werkzeugkupplungsglied 274, welches zum Abkuppeln des Bearbeitungswerkzeugs in Richtung des Pfeils 276 etwas zum Lagerkörper 158 hin verschiebbar ist. Das Werkzeugkupplungsglied 274 durchgreift das Gehäuse 162 teilweise und weist ein freies Ende 278 auf, welches in proximaler Richtung weist.

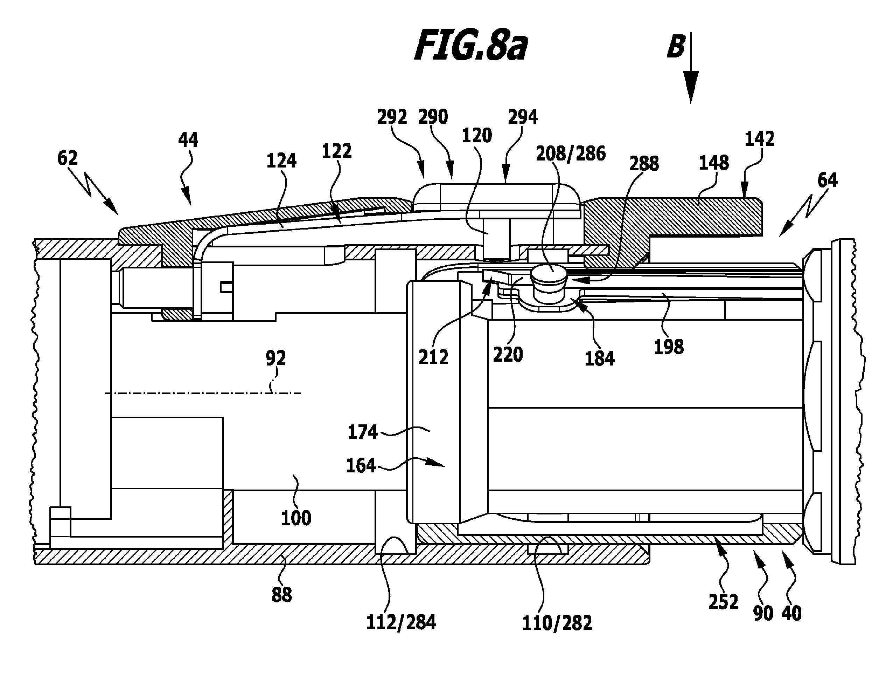

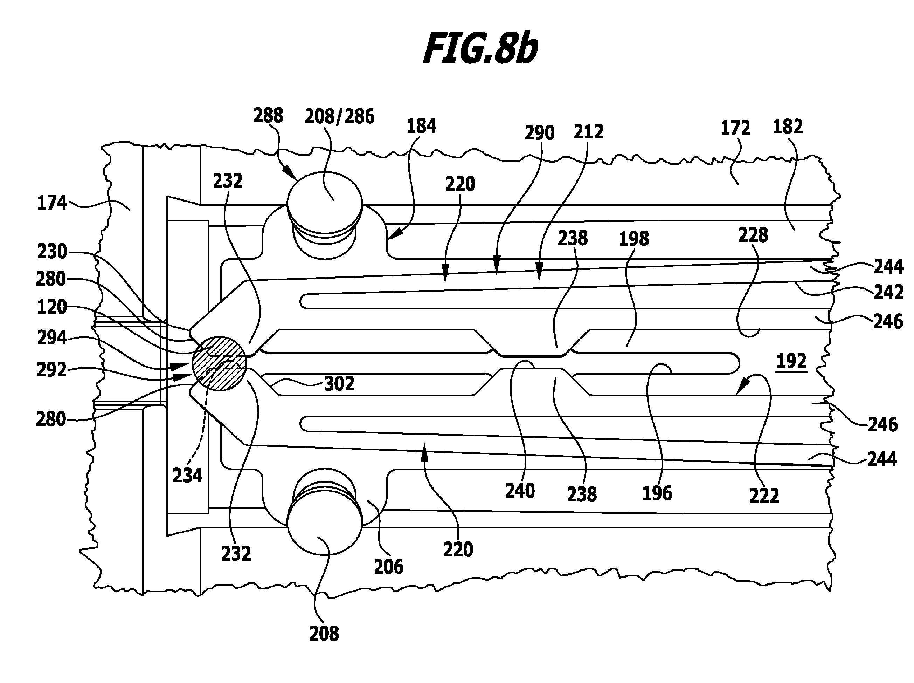

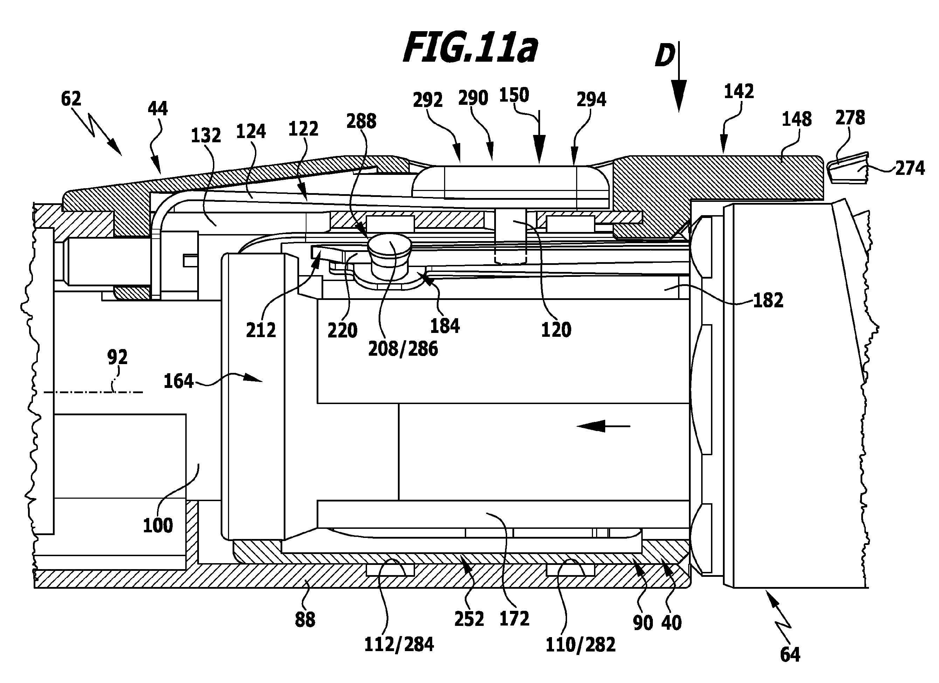

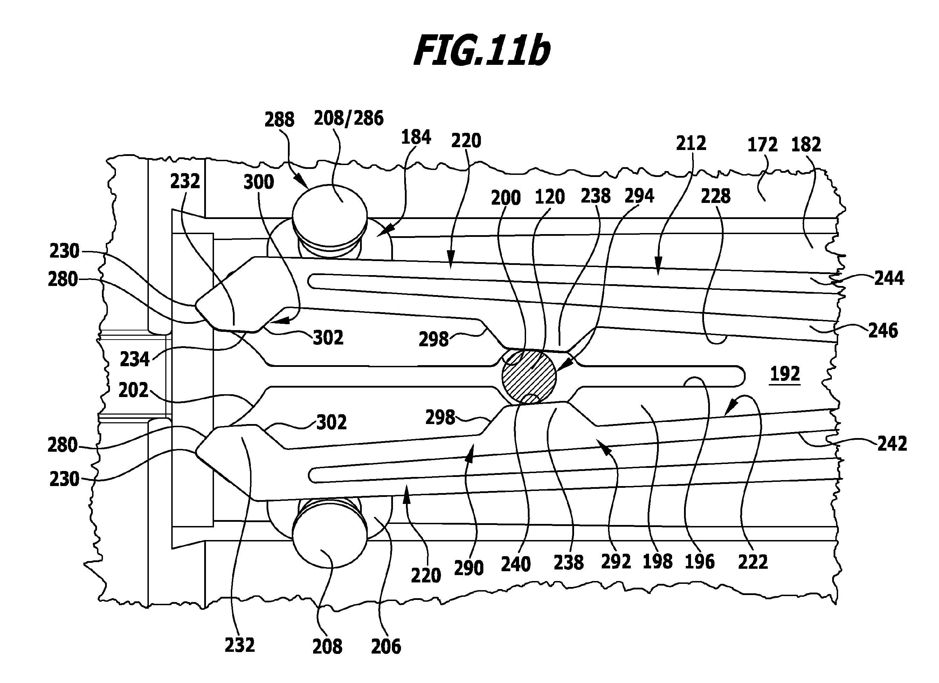

Die Funktionsweise des Kupplungssystems 60 wird nachfolgend in Verbindung mit den Figuren 7a bis 13 näher erläutert.

Ausgehend von der in Figur 2 dargestellten Trennstellung können die Kupplungsvorrichtungen 62 und 64 miteinander in Eingriff gebracht werden, so dass sie eine mechanische Kupplungsstellung einnehmen, wie sie schematisch in Figur 7a dargestellt ist. Dabei ist unerheblich, ob das Löseglied 120 die betätigte oder die unbetätigte Stellung einnimmt. In Figur 7a ist das Löseglied 120 in der betätigten Stellung dargestellt. Der Stift 118 ragt dann so weit über die Innenwand 108 vor, dass er an in proximaler Richtung weisenden schrägen Aufgleitkanten 280 der ersten Steuervorsprünge 232 aufgleitet und durch Vergrößerung des Spalts 234 zwischen diese eingreift und sie voneinander weg bewegt. Ein Abstand der ersten Steuervorsprünge 232 ist in dieser Stellung, die als Freigabestellung bezeichnet wird und schematisch in Figur 7b dargestellt ist, größer als in der in Figur 4 dargestellten Mitnahmestellung, die das Steuerelement 212 einnimmt, ohne dass äußere Kräfte auf es einwirken.

Die ersten Steuervorsprünge 232 sind zudem derart angeordnet, dass sie die Durchbrechung 202 in der Mitnahmestellung überdecken und in der Freigabestellung freigeben. Das Löseglied 120 kann somit in der Freigabestellung nicht in Kontakt mit dem Halteglied 192 treten. Damit können die Haltearme 198 in ihrer Grundstellung ihre maximal von der Längsachse 92 ausgefederte Stellung einnehmen. Ist der Kupplungszapfen 90 so weit in die Kupplungsachse 88 eingeführt, dass sich die Verriegelungsvorsprünge 208 auf Höhe der Ringnut 110 befinden, federn die Haltearme 198 in radialer Richtung von der Längsachse 92 weg aus und die Verriegelungsvorsprünge 208 greifen in die Ringnut 110 ein. Dies wird möglich, da beim Einführen des Kupplungszapfens 90 die Verriegelungsvorsprünge 208 an der Innenwand 108 aufgleiten und etwas in Richtung auf die Längsachse 92 hin verschwenkt wurden.

Die Ringnuten 110 und 112 bilden erste und zweite Verriegelungselemente 282 und 284, die Verriegelungsvorsprünge 208 dritte Verriegelungselemente 286 einer insgesamt mit dem Bezugszeichen 288 bezeichneten Verriegelungseinrichtung.

In der in Figur 7a dargestellten AUS-Stellung des Kupplungssystems 60 greifen die Kontaktstifte 260 und 263 in die korrespondierenden Steckbuchsen 102 und 105 ein. Damit wird ein Stromkreis geschlossen, in welchem der Kupplungskontakt 70 in Serie mit der Motorwicklung 79, der Motorwicklung 80, dem Widerstand 264 sowie dem Kupplungskontakt 73 geschaltet ist. Dies ist schematisch in Figur 16 dargestellt. In der AUS-Stellung kann durch Anlegen einer Spannung an die Leitungen 74 und 76 durch Bestimmen des dann fließenden Stroms bei Kenntnis der Widerstandswerte der Motorwicklungen 79 und 80 ein Widerstandswert des Widerstands 264 berechnet und aus dem so ermittelten Widerstandswert wiederum auf die Art des Elektromotors 82 geschlossen werden. Diese Bestimmung kann durch entsprechende Ausgestaltung beziehungsweise Programmierung des Steuergeräts 12 automatisch erfolgen.

Mit dem Steuergerät 12 kann dann der Elektromotor 82 in gewünschter Weise angesteuert werden, beispielsweise um ein Bearbeitungswerkzeug durch den Elektromotor 82 in Rotation zu versetzen.

Das Kupplungssystem 60 umfasst ferner eine Schaltstellungssicherungseinrichtung 290 zum mechanischen Sichern einer durch eine vom Kupplungssystem 60 umfassten und durch Zusammenwirken der beiden Kupplungsvorrichtungen 62 und 64 ausgebildeten Schalteinrichtung 292 in der beschriebenen AUS-Stellung sowie in einer AN-Stellung, in welcher jeder der Kontaktstifte 260, 261, 262 und 263 in die jeweils korrespondierende Steckbuchse 102, 103, 104 beziehungsweise 105 eingreift. Die Schaltstellungssicherungseinrichtung 290 umfasst die Verriegelungseinrichtung 288, welche ausgebildet ist zum mechanischen Verriegeln der die Kupplungsstellung einnehmenden Kupplungsvorrichtungen 62 und 64 in der beschriebenen AUS-Stellung sowie auch in der später noch beschriebenen AN-Stellung der Schalteinrichtung 292. Des Weiteren umfasst die Schaltstellungssicherungseinrichtung 290 eine insgesamt mit dem Bezugszeichen 294 bezeichnete Löseeinrichtung zum mechanischen Lösen der Verriegelungseinrichtung. Die Löseeinrichtung 294 ist ausgebildet zum außer Eingriff Bringen der Verriegelungselemente 282 und 286 von der AUS-Stellung in eine Schaltstellung, in welcher die Kupplungsvorrichtungen 62, 64 relativ zueinander in axialer Richtung bewegt werden könnten. Hierfür umfasst die Löseeinrichtung 294 das Löseglied 120. Bei dem in den Figuren dargestellten Kupplungssystem 60 ist das Löseglied 120 ausgebildet zum indirekten in Eingriff oder Kontakt bringen mit dem dritten Verriegelungselement 286 zum Überführen der Verriegelungseinrichtung 288 sowohl aus der AUS-Stellung als auch aus der AN-Stellung in die Schaltstellung.

Wird wie oben beschrieben der Kupplungszapfen 90 bei betätigtem Löseglied 20 in die Kupplungshülse 88 eingeführt, spreizt das Löseglied 120 die ersten Steuervorsprünge 232 auf, wodurch eine Betätigung des Halteglieds 192, in folge derer die beiden Verriegelungselemente 286 aus der Ringnut 110 herausgeführt würden, nicht möglich ist. Hierfür muss zuerst das Löseglied 120 freigegeben werden, damit das Steuerelement 212 von der Freigabestellung in die Mitnahmestellung übergehen kann. Die dritten Verriegelungselemente286, welche am Halteglied 192 angeordnet beziehungsweise gelagert sind, werden dann durch die Wirkung einer Vorspanneinrichtung 296 von der Längsachse 92 weg in radialer Richtung in die Ringnut 110 hinein bewegt. Die von der Verriegelungseinrichtung 288 umfasste Vorspanneinrichtung 296 dient zum Halten Verriegelungselemente 282 und 286 einerseits und 284 und 286 andererseits in der AUS-Stellung beziehungsweise in der AN-Stellung. Die Verriegelungselemente 282 und 286 beziehungsweise 284 und 286 können nur entgegen einer von der Vorspanneinrichtung 296 ausgeübten vorspannenden Kraft außer Eingriff gebracht werden. Die Vorspanneinrichtung 296 umfasst die Haltearme 198 und somit das Halteglied 192, welches federelastisch ausgebildet ist und ohne eine vom Löseglied 120 ausgeübte Kraft die Verriegelungselemente 286 von der Längsachse 292 weg bewegt oder in einer von der Längsachse 292 maximal entfernten Stelle hält.

Damit sichergestellt ist, dass beim miteinander in Eingriff Bringen der Kupplungsvorrichtungen 62 und 64 das Kupplungssystem 60 zunächst die AUS-Stellung einnimmt, unabhängig davon, ob das Löseglied 120 betätigt ist oder nicht, ist das Steuerelement 212 vorgesehen. Es ist von der Mitnahmestellung, in welcher das Löseglied 120 mittels des Steuerelements 212 indirekt mit dem dritten Verriegelungselement 266 in Kontakt oder Eingriff bringbar ist zum Überführen der Verriegelungseinrichtung 288 aus der AUS-Stellung oder aus der AN-Stellung in die Schaltstellung, in die Freigabestellung bringbar, in welcher das Löseglied 120 weder direkt noch indirekt mit dem dritten Verriegelungselement 286 hinreichend in Kontakt oder Eingriff bringbar ist zum Überführen der Verriegelungseinrichtung 288 aus der AUS-Stellung oder aus der AN-Stellung in die Schaltstellung. Diese Freigabestellung ist schematisch in den Figuren 7a und 7b dargestellt. Durch das Aufspreizen des Steuerelements 212 beim Eingreifen des Löseglieds 120 zwischen die ersten Steuervorsprünge 232 ist es dann, wenn das Löseglied 120 die betätigte Stellung einnimmt, nicht möglich, mit dem Löseglied 120 das Halteglied 192 in Richtung auf die Längsachse 92 zu bewegen, um dadurch die Verrieglungselemente 286 und 282 außer Eingriff zu bringen. Damit letzteres ermöglicht wird, muss zunächst das Löseglied 120 in seine unbetätigte Stellung überführt werden. Dies geschieht einfach dadurch, dass eine Bedienperson den Betätigungsvorsprung 138 freigibt, so dass der Lösehebel 122 in seine Ausgangsstellung zurückfedern kann, in welcher das Löseglied 120 etwas über eine Außenseite 136 des Führungskörpers 142 vorsteht.

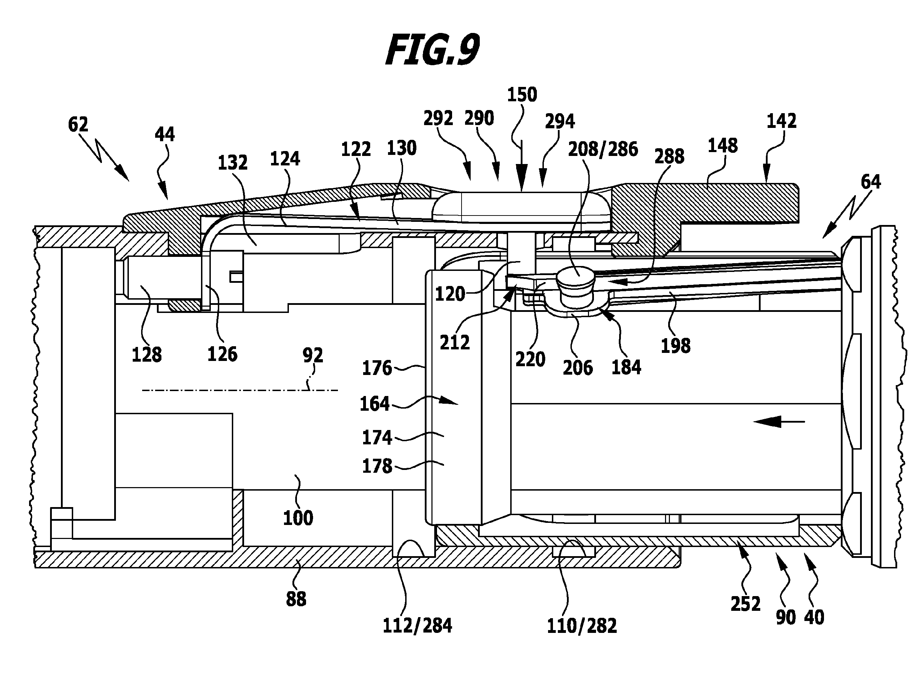

Durch das Herausbewegen des Löseglieds 120 aus dem Spalt 234 zwischen den ersten Steuerungsvorsprüngen 232 federn diese aufgrund der federelastischen Ausgestaltung der Steuerschenkel 244 in ihre angenäherte Stellung oder Mitnahmestellung zurück. In dieser überdecken die ersten Steuervorsprünge 232 die Durchbrechung 200. Wird das Löseglied 120 nun erneut betätigt, das heißt in Richtung auf die Längsachse 92 hin bewegt, so schlägt es zunächst an den beiden Steuervorsprüngen an, wie dies in Figur 9 schematisch dargestellt ist, und nimmt bei einer Verschwenkbewegung der Steuerarme 220 in Richtung auf die Längsachse 92 die Haltearme 198 mit, wodurch die Verriegelungselemente 286 aus der Ringnut 110 herausbewegt werden. Das Kupplungssystem 60 befindet sich nun in der Schaltstellung. Die beiden Kupplungsvorrichtungen 62 und 64 können nun entweder wieder zurück in die Trennstellung überführt werden, in dem sie auseinander gezogen werden, oder noch weiter zusammengeschoben werden, um die Schalteinrichtung 292 von der AUS-Stellung in die AN-Stellung zu überführen.

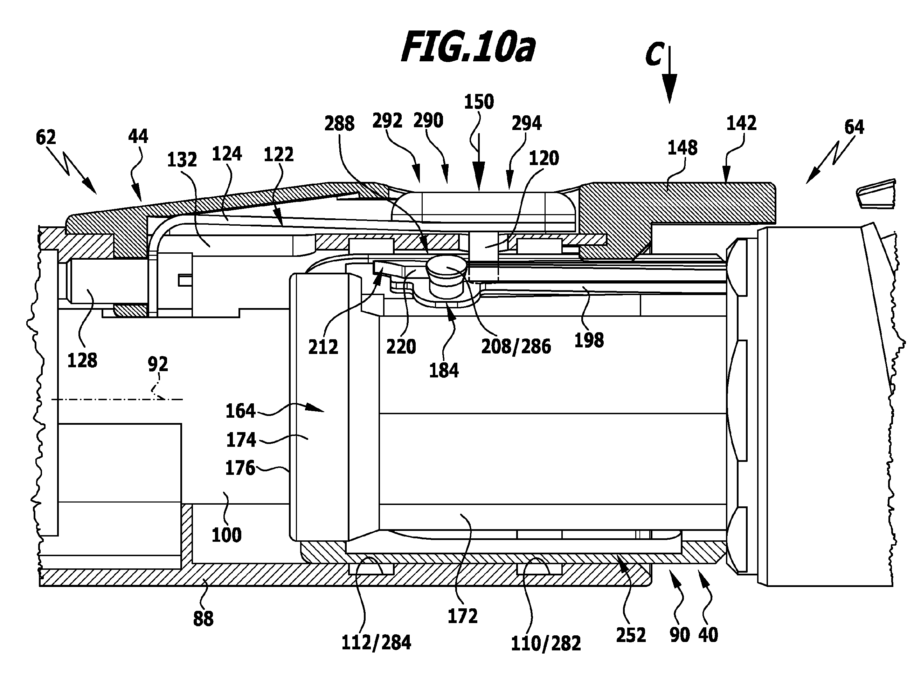

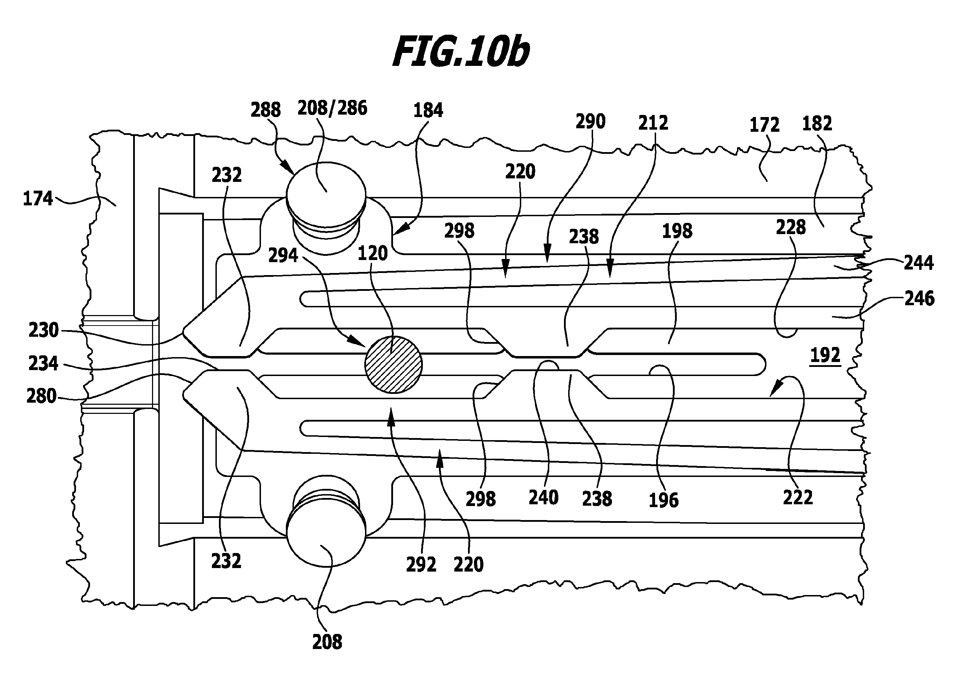

Werden die Kupplungsvorrichtungen 62 und 64, wie in den Figuren 10a und 10b schematisch dargestellt, weiter aufeinander zu bewegt, so federt bei betätigtem Löseglied 120 das Halteglied 192 radial nach außen, sobald das Löseglied 120 in den Bereich zwischen den ersten Steuervorsprüngen 232 und den zweiten Steuervorsprüngen 238 eingreift. Wird das Löseglied 120 betätigt gehalten und die Kupplungsvorrichtungen 62 und 64 weiter aufeinander zu bewegt, dann schlägt das Löseglied an in proximaler Richtung weisenden Aufgleitkanten 298 der zweiten Steuervorsprünge 238 an und schiebt sich zwischen diese in den Spalt 240 hinein, wodurch die Steuerarme 220 wiederum aufgespreizt und die zweiten Steuervorsprünge 238 auseinanderbewegt werden unter gleichzeitiger Vergrößerung eines Abstands zwischen ihnen und somit auch einer Breite des Spalts 240. Das Steuerelement 212 nimmt nun wieder die Freigabestellung in, in welcher sich das Löseglied 120 direkt über der Durchbrechung 200 befindet und somit nicht mit dem Halteglied 192 und damit indirekt mit den dritten Verriegelungselementen 286 in Eingriff gelangen kann. Genau in der Stellung, in der das Löseglied 120 in den Spalt 240 eingreift, befinden sich die Verriegelungselemente 286 auf Höhe der Ringnut 112 und werden durch die Vorspanneinrichtung 296 somit mit dem zweiten Verriegelungselement 284 in Eingriff gebracht. Mit anderen Worten verrastet das Kupplungssystem 60 automatisch in der AN-Stellung, sobald sich die Verriegelungsvorsprünge 208 axial auf Höhe der Ringnut 212 befinden.

In der AN-Stellung stehen auch die Kupplungskontakte 67 und 71 einerseits sowie die Kupplungskontakte 68 und 72 andererseits in Eingriff. Die AN-Stellung ist schematisch in Figur 16c dargestellt. In der AN-Stellung können die Wicklungen 78, 79 und 80 des Elektromotors 82 mittels des Steuergeräts 12 in gewünschter Weise bestromt werden, um den Elektromotor 82 und ein gegebenenfalls mit ihm gekuppeltes Bearbeitungswerkzeug in Rotation zu versetzen.

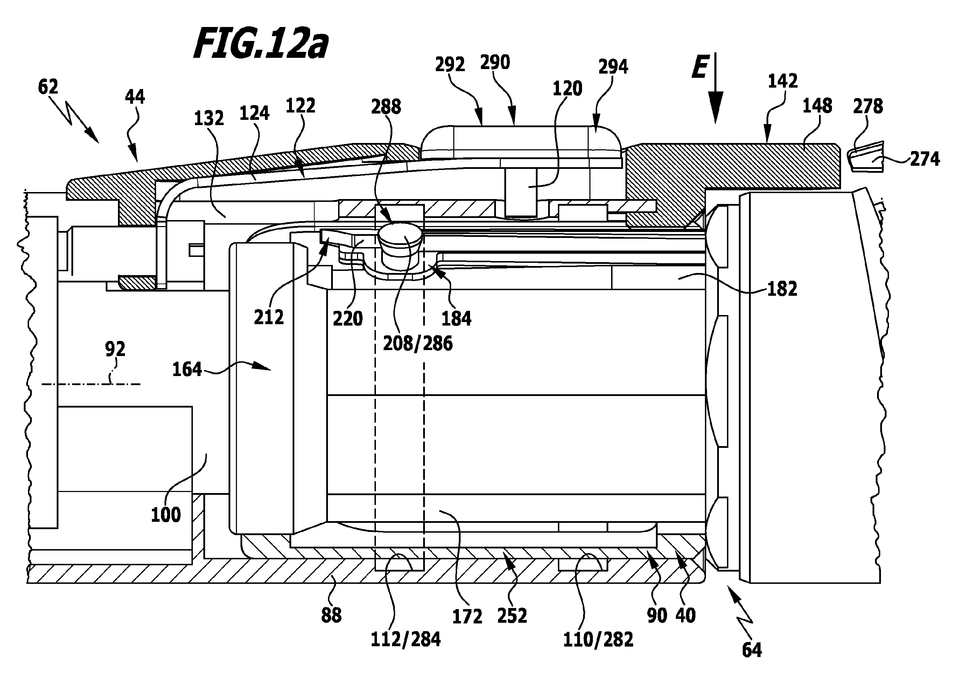

Des Weiteren hat die Verriegelungseinrichtung 288 noch eine weitere Funktion. In der AN-Stellung des Kupplungssystems 60, wie schematisch in Figur 12a dargestellt, bildet das Blockierglied 148 einen Anschlag für das Werkzeugkupplungsglied 274 und verhindert eine Bewegung desselben in proximaler Richtung. Ist ein Bearbeitungswerkzeug mit dem Handstück 14 gekoppelt und das Werkzeugkupplungsglied 274 in seiner distalsten Stellung, also nicht zurückgezogen, nimmt die Werkzeugkupplungseinrichtung 272 die Werkzeugkupplungsstellung ein. Ein Zurückziehen in proximaler Richtung in eine Werkzeugtrennstellung, ist somit nur möglich, wenn sich das Kupplungssystem 60 nicht in der AN-Stellung befindet, also ein Abstand zwischen dem Blockierglied 148 und einem proximalen Ende des Werkzeugkupplungsglieds 274 ausreichend groß ist, so dass letzteres von der Werkzeugkupplungsstellung in die Werkzeugtrennstellung bewegt werden kann. Diese Sicherungsfunktion des Kupplungssystems 60 verhindert, dass eine Bedienperson dann, wenn sich das Kupplungssystem 60 in der AN-Stellung befindet, versehentlich das Werkzeugkupplungsglied 274 betätigen und so ein unerwünschtes Entkuppeln oder Lösen eines mit dem Handstück 14 gekuppelten Bearbeitungswerkzeugs verhindern kann.

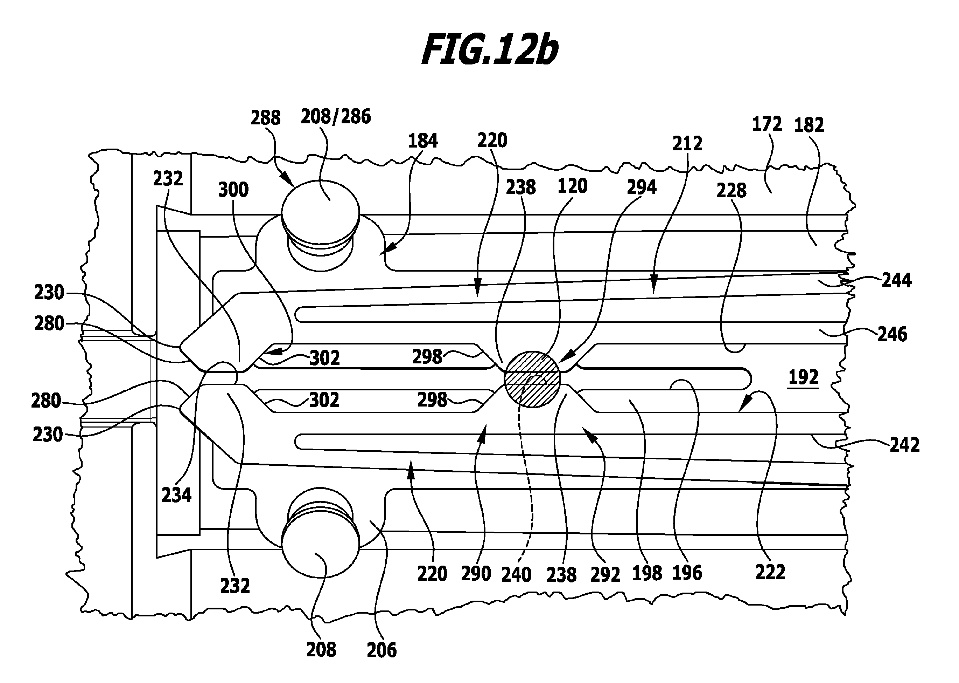

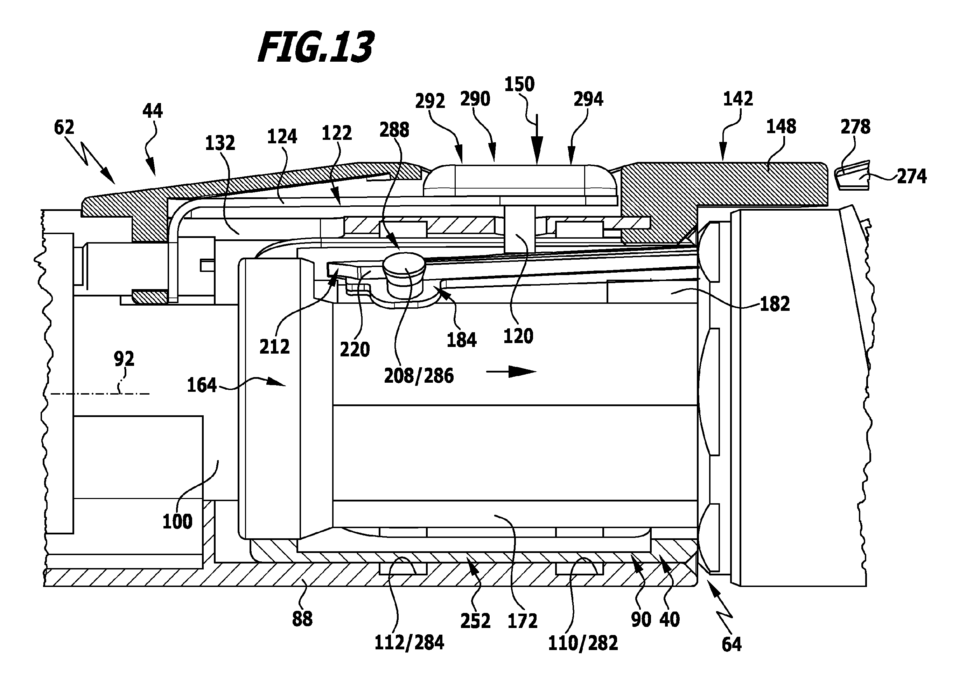

Nimmt das Kupplungssystem 60 die AN-Stellung ein, so kann es nur aktiv von der AN-Stellung zunächst in die Schaltstellung überführt werden, wenn das Löseglied 120 mindestens einmal in seine unbetätigte Stellung überführt wurde. Dies ist schematisch in Figuren 12a und 12b dargestellt. Zum Entriegeln, das heißt zum außer Eingriff Bringen der Verriegelungsvorsprünge 208 und der Ringnut 112 muss dann das Löseglied 120 wieder in Richtung auf die Längsachse 92 hin bewegt werden. Durch das Überführen des Löseglieds 120 von der betätigten in die unbetätigte Stellung, konnten die zweiten Steuervorsprünge 238 durch die Wirkung der eine Rückstelleinrichtung 300 bildenden Steuerarme 220 wieder aufeinander zu bewegt werden, wodurch der Spalt 240 minimiert wurde. Eine Bewegung des Löseglieds 120 in Richtung auf das nun wie in Figur 12b schematisch dargestellt die Mitnahmestellung einnehmenden Steuerelements 212 gestattet es, dies quasi als Mitnehmer für das Halteglied 192 zu nutzen, so dass in Folge einer Verschwenkung der zweiten Steuervorsprünge 238 in Richtung auf die Längsachse 92 hin die Haltearme 198 ebenfalls in Richtung auf die Längsachse 92 hin verschwenkt werden, wodurch die Verriegelungsvorsprünge 208 und die Ringnut 212 außer Eingriff gelangen. Dies ist schematisch in Figur 13 dargestellt. Die Kupplungsvorrichtungen 62 und 64 können nun wieder auseinander gezogen werden.

Allerdings ist beim Auseinanderziehen ebenfalls ein Überfahren der AUS-Stellung, wie bereits beim Zusammenführen der Kupplungsvorrichtungen 62 und 64 nicht möglich. Ist das Löseglied 120 beim Auseinanderziehen der Kupplungsvorrichtungen 62 und 64 betätigt, gelangt es zunächst mit in distaler Richtung weisenden schrägen Aufgleitkanten 302 der ersten Steuervorsprünge 232 in Kontakt und spreizt diese wiederum auf. Sobald die Verriegelungsvorsprünge 208 sich auf Höhe der Ringnut 110 befinden, bewirkt die Vorspanneinrichtung 296 ein Verrasten derselben miteinander. Auf diese Weise kann verhindert werden, dass beim Übergang von der AN-Stellung in die AUS-Stellung die Kupplungsvorrichtungen 62 und 64 unabsichtlich voneinander gelöst werden. Wird dies jedoch gewünscht, muss zunächst das Löseglied 120 wieder in die unbetätigte Stellung überführt werden. Durch Freigeben des Spalts 234 federn die ersten Steuervorsprünge 232 wieder zurück und aufeinander zu, nehmen also wieder die Mitnahmestellung ein, und ermöglichen es, dass in Folge einer erneuten Betätigung des Löseglieds 120 die Verriegelungsvorsprünge 208 und die Ringnut 110 wieder außer Eingriff gelangen können. Das Kupplungssystem 60 kann nun entweder in seine Trennstellung oder wieder zurück in die AN-Stellung überführt werden.

Durch die besondere Ausgestaltung des Kupplungssystems 10 kann also auf einfache Weise ein elektromechanisches Schaltelement in Form der Spalteinrichtung 292 ausgebildet werden.

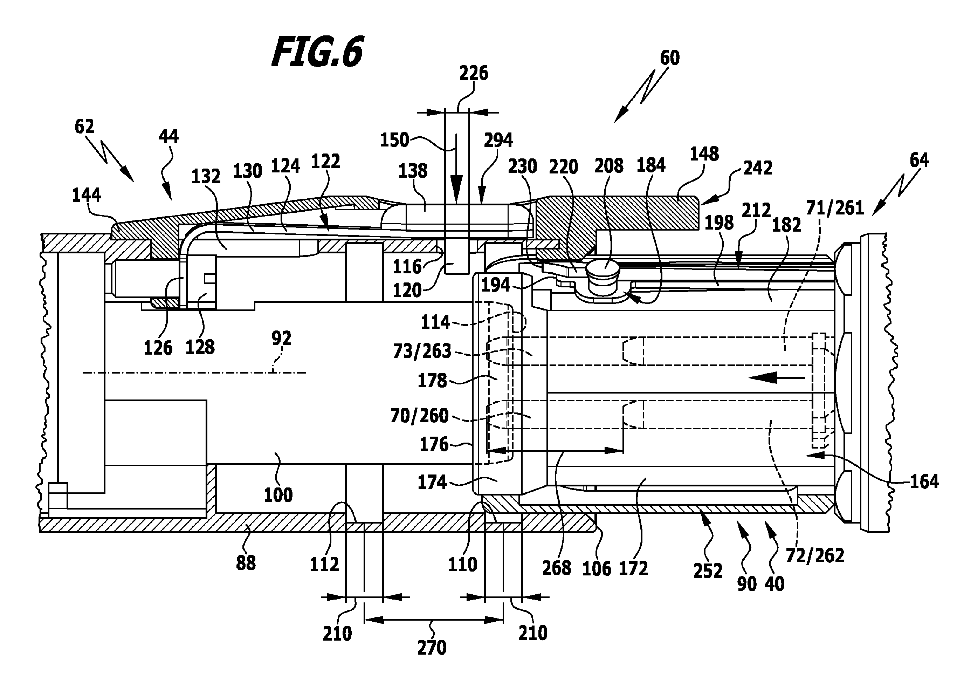

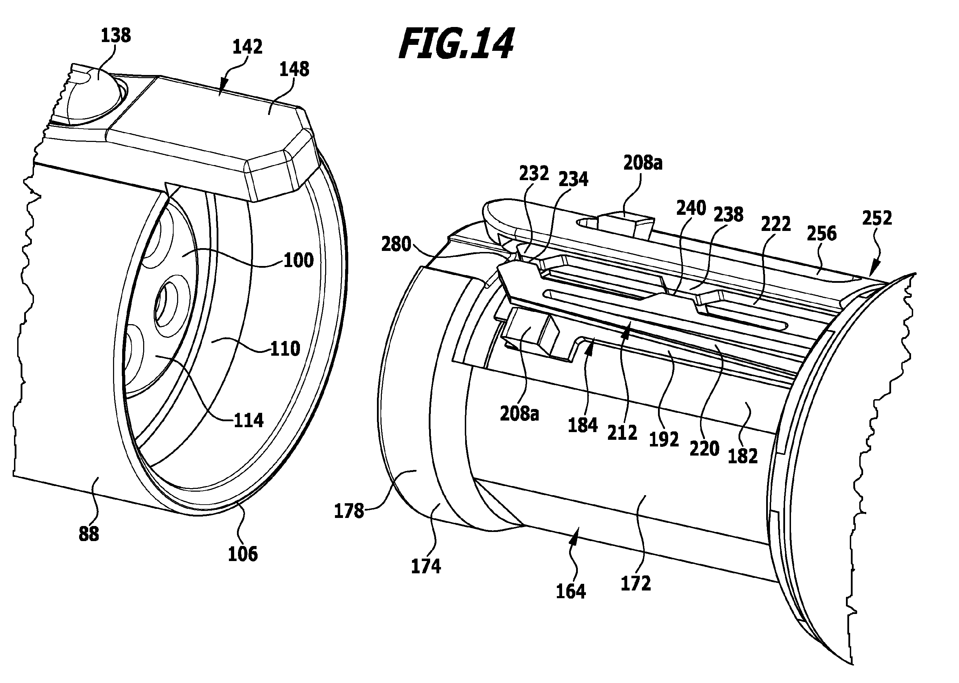

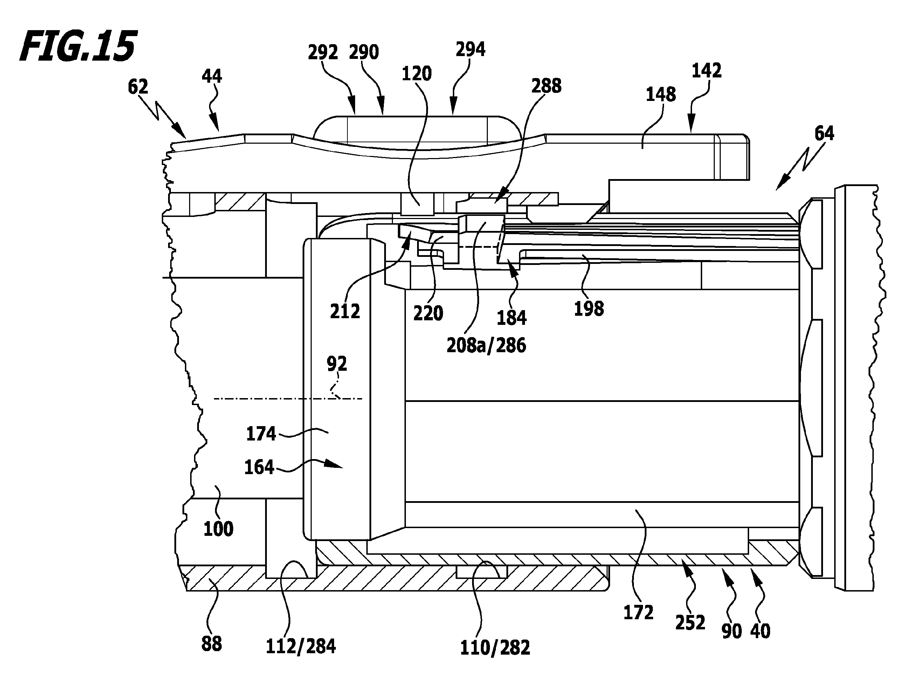

Eine leicht modifizierte Ausfertigungsform des Kupplungssystems 60 ist schematisch in den Figuren 14 und 15 teilweise dargestellt. Es unterscheidet sich vom oben beschriebenen Kupplungssystem 60 lediglich durch die Form der Verriegelungsvorsprünge 208. Diese sind bei dem in den Figuren 14 und 15 dargestellten Ausführungsbeispiel im Wesentlichen quaderförmig ausgebildet im Gegensatz zur rotationssymmetrischen Ausgestaltung bei dem in den Figuren 2 bis 13 dargestellten Ausführungsbeispiel.

Das Kupplungssystem ist vorzugsweise ausschließlich aus Materialien hergestellt, welche für eine Heißdampfsterilisation geeignet sind.

## Patentansprüche

1. Chirurgisches Kupplungssystem (60) umfassend eine erste chirurgische Kupplungsvorrichtung (62) und eine zweite chirurgische Kupplungsvorrichtung (64) mit jeweils mindestens zwei mechanisch miteinander in Eingriff bringbaren elektrischen Kupplungskontakten (66, 67, 68, 69, 70, 71, 72, 73, 74), wobei die erste und die zweite Kupplungsvorrichtung (62, 64) in einer Trennstellung vollständig voneinander getrennt sind und in einer mechanischen Kupplungsstellung mechanisch miteinander in Eingriff stehen, **gekennzeichnet durch** eine elektrische Schalteinrichtung (292), welche in der Kupplungsstellung von einer AUS-Stellung, in welcher mindestens ein erster elektrischer Kupplungskontakt (67, 68) der ersten Kupplungsvorrichtung (62) und mindestens ein erster elektrischer Kupplungskontakt (71, 72) der zweiten Kupplungsvorrichtung (64) außer Eingriff stehen, in eine AN-Stellung, in welcher der mindestens eine erste elektrische Kupplungskontakt (67, 68) der ersten Kupplungsvorrichtung und der mindestens eine erste elektrische Kupplungskontakt (71, 72) der zweiten Kupplungsvorrichtung elektrisch leitend in Kontakt oder Eingriff stehen, bringbar ist und/oder umgekehrt.

2. Chirurgisches Kupplungssystem nach Anspruch 1, **dadurch gekennzeichnet, dass** die erste und die zweite Kupplungsvorrichtung (62, 64) jeweils mindestens einen zweiten elektrischen Kupplungskontakt (66, 69, 70, 73) umfassen und dass die zweiten Kupplungskontakte (66, 69, 70, 73) in der AUS-Stellung und in der AN-Stellung miteinander elektrisch leitend in Kontakt oder Eingriff stehen, wobei insbesondere die ersten elektrischen Kupplungskontakte (71, 72) kürzer sind als die zweiten elektrischen Kupplungskontakte (70, 73).

3. Chirurgisches Kupplungssystem nach Anspruch 1 oder 2, **dadurch gekennzeichnet, dass** die eine der beiden Kupplungsvorrichtungen (62) mindestens ein erstes Kupplungselement (84) umfasst, dass die andere der beiden Kupplungsvorrichtung (64) mindestens ein zweites Kupplungselement (86) umfasst, welches korrespondierend zum mindestens einen ersten Kupplungselement (84) ausgebildet ist, und dass das mindestens eine erste und das mindestens eine zweite Kupplungselement (86) in der Kupplungsstellung miteinander in Eingriff stehen.

4. Chirurgisches Kupplungssystem nach einem der voranstehenden Ansprüche, **gekennzeichnet durch** eine Schaltstellungssicherungseinrichtung (290) zum mechanischen Sichern der Schalteinrichtung (292) in der AN-Stellung und in der AUS-Stellung.

5. Chirurgisches Kupplungssystem nach Anspruch 4, **dadurch gekennzeichnet, dass** die Schaltstellungssicherungseinrichtung (290) eine Verriegelungseinrichtung (288) zum mechanischen Verriegeln der die Kupplungsstellung einnehmenden ersten und zweiten Kupplungsvorrichtungen (62, 64) in der AN-Stellung und in der AUS-Stellung und eine Löseeinrichtung (294) zum mechanischen Lösen der Verriegelungseinrichtung (288) umfasst.

6. Chirurgisches Kupplungssystem nach Anspruch 5, **dadurch gekennzeichnet, dass** die Verriegelungseinrichtung (288) erste und zweite Verriegelungselemente (282, 284) sowie ein drittes Verriegelungselement (286) umfasst, dass die ersten und zweiten Verriegelungselemente (282, 284) an der einen der beiden Kupplungsvorrichtungen (62, 64) angeordnet oder ausgebildet sind, dass das dritte Verriegelungselement (286) an der anderen der beiden Kupplungsvorrichtungen (62, 64) angeordnet oder ausgebildet ist und dass das erste und das dritte Verriegelungselement (284, 286) in der AN-Stellung in Eingriff stehen und dass das zweite und das dritte Verriegelungselement (282, 286) in der AUS-Stellung in Eingriff stehen.

7. Chirurgisches Kupplungssystem nach Anspruch 6, **dadurch gekennzeichnet, dass** die Verriegelungseinrichtung (288) eine Vorspanneinrichtung (296) umfasst zum Halten der Verriegelungselemente (282, 284, 286) in der AN-Stellung beziehungsweise in der AUS-Stellung.

8. Chirurgisches Kupplungssystem nach Anspruch 6 oder 7, **dadurch gekennzeichnet, dass** die Löseeinrichtung (294) ein bewegbar gelagertes Löseglied (120) umfasst, welches von einer unbetätigten Stellung in eine betätigte Stellung bringbar ist und/oder umgekehrt

9. Chirurgisches Kupplungssystem nach Anspruch 8, **dadurch gekennzeichnet, dass** das Löseglied (120) mit dem dritten Verriegelungselement (286) direkt oder indirekt in Eingriff oder Kontakt bringbar ist zum Überführen der Verriegelungseinrichtung (288) aus der AN-Stellung oder aus der AUS-Stellung in die Schaltstellung.

10. Chirurgisches Kupplungssystem nach Anspruch 9, **dadurch gekennzeichnet, dass** die Löseeinrichtung (294) mindestens ein mechanisches Steuerelement (212) umfasst, welches von einer Mitnahmestellung, in welcher das Löseglied (120) mittels des Steuerelements (212) direkt oder indirekt mit dem dritten Verriegelungselement (286) in Kontakt oder Eingriff bringbar ist zum Überführen der Verriegelungseinrichtung (288) aus der AN-Stellung oder aus der AUS-Stellung in die Schaltstellung, in eine Freigabestellung bringbar ist, in welcher das Löseglied (120) weder direkt noch indirekt mit dem dritten Verriegelungselement (286) hinreichend in Kontakt oder Eingriff bringbar ist zum Überführen der Verriegelungseinrichtung (288) aus der AN-Stellung oder aus der AUS-Stellung in die Schaltstellung.

11. Chirurgisches Kupplungssystem nach Anspruch 10, **dadurch gekennzeichnet, dass** die Löseeinrichtung (294) derart ausgebildet ist, dass das Löseglied (120) in der betätigten Stellung das Steuerelement (212) von der Mitnahmestellung in die Freigabestellung überführt zum Verhindern, dass das Löseglied (120) direkt oder indirekt mit dem dritten Verriegelungselement (286) in Kontakt oder Eingriff bringbar ist zum Überführen der Verriegelungseinrichtung (288) aus der AN-Stellung oder aus der AUS-Stellung in die Schaltstellung.

12. Chirurgisches Kupplungssystem nach Anspruch 10 oder 11, **dadurch gekennzeichnet, dass** die Löseeinrichtung (294) derart ausgebildet ist, dass nur durch Überführen des Löseglieds (120) von der betätigten Stellung in die unbetätigte Stellung das Steuerelement (212) von der Freigabestellung in die Mitnahmestellung überführbar ist.

13. Chirurgisches Kupplungssystem nach einem der Ansprüche 10 bis 12, **dadurch gekennzeichnet, dass** die Löseeinrichtung (294) derart ausgebildet ist, dass das Löseglied (120) nur in der Mitnahmestellung direkt oder indirekt mit dem dritten Verriegelungselement (286) in Kontakt oder Eingriff bringbar ist zum Überführen der Verriegelungseinrichtung (288) aus der AN-Stellung oder aus der AUS-Stellung in die Schaltstellung.

14. Chirurgisches Kupplungssystem nach einem der Ansprüche 10 bis 13, **dadurch gekennzeichnet, dass** die Löseeinrichtung (294) eine Rückstelleinrichtung (220) umfasst zum automatischen Überführen des Steuerelements (212) von der Freigabestellung in die Mitnahmestellung, sobald das Löseglied (120) von der betätigten Stellung in die unbetätigte Stellung überführt wird.

15. Chirurgisches Antriebssystem (10) umfassend mindestens ein chirurgisches Handstück (14) mit einem Antrieb in Form eines Elektromotors (82) und mindestens eine elektrische Versorgungsleitung (20), welche Versorgungsleitung (20) ein erstes, mit dem mindestens einen Handstück (14) lösbar verbindbares Ende und ein zweites, mit einer Steuer- und/oder Regelungsvorrichtung (12) zum Steuern und/oder Regeln des Elektromotors (82) verbundenes oder lösbar verbindbares Ende aufweist, **gekennzeichnet durch** ein Kupplungssystem (60) zum elektrischen und mechanischen Verbinden des Handstücks (14) und der Versorgungsleitung (20) nach einem der voranstehenden Ansprüche.

## Claims

1. A surgical coupling system (60) comprising a first surgical coupling device (62) and a second surgical coupling device (64) each having at least two electrical coupling contacts (66, 67, 68, 69, 70, 71, 72, 73, 74) that are moveable mechanically into engagement with one another, wherein the first and the second coupling device (62, 64) are completely separated from each other in a separated position and are mechanically in engagement with one another in a mechanical coupling position, **characterized by** an electrical switching device (292) which, in the coupling position, is moveable from an OFF position, in which at least one first electrical coupling contact (67, 68) of the first coupling device (62) and at least one first electrical coupling contact (71, 72) of the second coupling device (64) are out of engagement, into an ON position in which the at least one first electrical coupling contact (67, 68) of the first coupling device and the at least one first electrical coupling contact (71, 72) of the second coupling device are in electrically conductive contact or engagement, and/or vice versa.

2. A surgical coupling system in accordance with Claim 1, **characterised in that** the first and the second coupling device (62, 64) each comprise at least one second electrical coupling contact (66, 69, 70, 73), and **in that** the second coupling contacts (66, 69, 70, 73) are in electrically conductive contact or engagement with one another in the OFF position and in the ON position, wherein, in particular, the first electrical coupling contacts (71, 72) are shorter than the second electrical coupling contacts (70, 73)..

3. A surgical coupling system in accordance with Claim 1 or 2, **characterised in that** the one of the two coupling devices (62) comprises at least one first coupling element (84), **in that** the other one of the two coupling devices (64) comprises at least one second coupling element (86) which is formed in correspondence with the at least one first coupling element (84), and **in that** the at least one first and the at least one second coupling element (86) are in engagement with one another in the coupling position.

4. A surgical coupling system in accordance with any of the preceding Claims, **characterized by** a switching position securing device (290) for mechanically securing the switching device (292) in the ON position and in the OFF position.

5. A surgical coupling system in accordance with Claim 4, **characterised in that** the switching position securing device (290) comprises a locking device (288) for mechanically locking the first and second coupling devices (62, 64) adopting the coupling position in the ON position and in the OFF position and a releasing device (294) for mechanically releasing the locking device (288).

6. A surgical coupling system in accordance with Claim 5, **characterised in that** the locking device (288) comprises first and second locking elements (282, 284) and also a third locking element (286), **in that** the first and second locking elements (282, 284) are arranged or formed on the one of the two coupling devices (62, 64), **in that** the third locking element (286) is arranged or formed on the other one of the two coupling devices (62, 64), and **in that** the first and the third locking element (284, 286) are in engagement in the ON position and **in that** the second and the third locking element (282, 286) are in engagement in the OFF position.

7. A surgical coupling system in accordance with Claim 6, **characterised in that** the locking device (288) comprises a biasing device (296) for holding the locking elements (282, 284, 286) in the ON position or in the OFF position.

8. A surgical coupling system in accordance with Claim 6 or 7, **characterised in that** the releasing device (294) comprises a movably mounted release member (120) which is moveable from an unactuated position into an actuated position and/or vice versa.

9. A surgical coupling system in accordance with Claim 8, **characterised in that** the release member (120) is moveable directly or indirectly into engagement or contact with the third locking element (286) to transfer the locking device (288) from the ON position or from the OFF position into the switching position.

10. A surgical coupling system in accordance with Claim 9, **characterised in that** the releasing device (294) comprises at least one mechanical control element (212) which is moveable from a driving position, in which the release member (120) is moveable directly or indirectly into contact or engagement with the third locking element (286) by means of the control element (212) to transfer the locking device (288) from the ON position or from the OFF position into the switching position, into a release position in which the release member (120) is moveable neither directly nor indirectly into contact or engagement with the third locking element (286) to an extent sufficient for transferring the locking device (288) from the ON position or from the OFF position into the switching position.

11. A surgical coupling system in accordance with Claim 10, **characterised in that** the releasing device (294) is configured in such a manner that the release member (120) in the actuated position transfers the control element (212) from the driving position into the release position for preventing the release member (120) being moveable directly or indirectly into contact or engagement with the third locking element (286) to transfer the locking device (288) from the ON position or from the OFF position into the switching position.

12. A surgical coupling system in accordance with Claim 10 or 11, **characterised in that** the releasing device (294) is configured in such a manner that the control element (212) is transferable from the release position into the driving position only by transferring the release member (120) from the actuated position into the unactuated position.

13. A surgical coupling system in accordance with any of the Claims 10 to 12, **characterised in that** the releasing device (294) is configured in such a manner that only in the driving position is the release member (120) moveable directly or indirectly into contact or engagement with the third locking element (286) to transfer the locking device (288) from the ON position or from the OFF position into the switching position.

14. A surgical coupling system in accordance with any of the Claims 10 to 13, **characterised in that** the releasing device (294) comprises a restoring device (220) for automatically transferring the control element (212) from the release position into the driving position as soon as the release member (120) is transferred from the actuated position into the unactuated position.

15. A surgical drive system (10) comprising at least one surgical hand-piece (14) having a drive in the form of an electric motor (82) and at least one electrical supply line (20), which supply line (20) comprises a first end that is connectable to the at least one hand-piece (14) in releasable manner and a second end that is connected or connectable in releasable manner to a control and/or regulating device (12) for controlling and/or regulating the electric motor (82), **characterised by** a coupling system (60) for the electrical and mechanical connection of the hand-piece (14) and the supply line (20) in accordance with any of the preceding Claims.

## Revendications

1. Système d'accouplement chirurgical (60) comprenant un premier dispositif d'accouplement chirurgical (62) et un deuxième dispositif d'accouplement chirurgical (64) comportant chacun respectivement au moins deux contacts électriques d'accouplement (66, 67, 68, 69, 70, 71, 72, 73, 74) pouvant être amenés en prise mécanique réciproque, le premier et le deuxième dispositif d'accouplement (62, 64) étant totalement séparés l'un de l'autre dans une position de séparation, et étant en prise mécanique réciproque dans une position d'accouplement mécanique,
**caractérisé par** un dispositif de commutation électrique (292), qui, dans la position d'accouplement, peut être amené d'une position ARRÊT, dans laquelle au moins un premier contact électrique d'accouplement (67, 68) du premier dispositif d'accouplement (62) et au moins un premier contact électrique d'accouplement (71, 72) du deuxième dispositif d'accouplement (64) sont hors de prise, à une position MARCHE, dans laquelle ledit au moins un premier contact électrique d'accouplement (67, 68) du premier dispositif d'accouplement et ledit au moins un premier contact électrique d'accouplement (71, 72) du deuxième dispositif d'accouplement sont en contact ou en prise de manière électriquement conductrice, et/ou inversement.

2. Système d'accouplement chirurgical selon la revendication 1, **caractérisé en ce que** le premier et le deuxième dispositif d'accouplement (62, 64) comportent chacun respectivement au moins un deuxième contact électrique d'accouplement (66, 69, 70, 73), et **en ce que** lesdits deuxièmes contacts électriques d'accouplement (66, 69, 70, 73) sont en contact ou en prise de manière électriquement conductrice dans la position ARRÊT et dans la position MARCHE, lesdits premiers contacts électriques d'accouplement (71, 72), en particulier, étant plus courts que les deuxièmes contacts électriques d'accouplement (70, 73).

3. Système d'accouplement chirurgical selon la revendication 1 ou la revendication 2, **caractérisé en ce que** l'un (62) des deux dispositifs d'accouplement comprend au moins un premier élément d'accouplement (84), **en ce que** l'autre (64) des deux dispositifs d'accouplement comprend au moins un deuxième élément d'accouplement (86), qui est configuré de manière correspondante au premier élément d'accouplement (84), et **en ce que** ledit au moins un premier et ledit au moins un deuxième élément d'accouplement (86) sont en prise réciproque dans la position d'accouplement.

4. Système d'accouplement chirurgical selon l'une des revendications précédentes, **caractérisé par** un dispositif d'arrêt de sécurisation de position de commutation (290) destiné à assurer un arrêt de sécurisation mécanique du dispositif de commutation (292) dans la position MARCHE et dans la position ARRÊT.

5. Système d'accouplement chirurgical selon la revendication 4, **caractérisé en ce que** le dispositif d'arrêt de sécurisation de position de commutation (290) comprend un dispositif de verrouillage (288) pour verrouiller mécaniquement les premier et deuxième dispositifs d'accouplement (62, 64) prenant la position d'accouplement, dans la position MARCHE et dans la position ARRÊT, et un dispositif de déblocage (294) pour débloquer mécaniquement le dispositif de verrouillage (288).

6. Système d'accouplement chirurgical selon la revendication 5, **caractérisé en ce que** le dispositif de verrouillage (288) comprend des premier et deuxième éléments de verrouillage (282, 284) ainsi qu'un troisième élément de verrouillage (286), **en ce que** les premier et deuxième éléments de verrouillage (282, 284) sont agencés ou formés sur l'un des deux dispositifs d'accouplement (62, 64), **en ce que** le troisième élément de verrouillage (286) est agencé ou formé sur l'autre des deux dispositifs d'accouplement (62, 64), et **en ce que** le premier et le troisième éléments de verrouillage (284, 286) sont en prise réciproque dans la position MARCHE, et **en ce que** le deuxième et le troisième éléments de verrouillage (282, 286) sont en prise réciproque dans la position ARRÊT.

7. Système d'accouplement chirurgical selon la revendication 6, **caractérisé en ce que** le dispositif de verrouillage (288) comprend un dispositif de précontrainte (296) pour assurer le maintien des éléments de verrouillage (282, 284, 286) dans la position MARCHE, respectivement dans la position ARRÊT.

8. Système d'accouplement chirurgical selon la revendication 6 ou la revendication 7, **caractérisé en ce que** le dispositif de déblocage (294) comprend un organe de déblocage (120) monté mobile, qui peut être amené d'une position non actionnée à une position actionnée, et/ou inversement.

9. Système d'accouplement chirurgical selon la revendication 8, **caractérisé en ce que** l'organe de déblocage (120) peut être amené directement ou indirectement en prise ou en contact avec le troisième élément de verrouillage (286), en vue du transfert du dispositif de verrouillage (288) de la position MARCHE ou de la position ARRÊT à la position de commutation.

10. Système d'accouplement chirurgical selon la revendication 9, **caractérisé en ce que** le dispositif de déblocage (294) comprend au moins un élément de commande mécanique (212), qui peut être amené d'une position d'entraînement, dans laquelle l'organe de déblocage (120) peut être amené au moyen de l'élément de commande (212), directement ou indirectement en contact ou en prise avec le troisième élément de verrouillage (286) pour le transfert du dispositif de verrouillage (288) de la position MARCHE ou de la position ARRÊT à la position de commutation, à une position de dégagement, dans laquelle l'organe de déblocage (120) ne peut être amené, ni directement, ni indirectement, suffisamment en contact ou en prise avec le troisième élément de verrouillage (286) pour le transfert du dispositif de verrouillage (288) de la position MARCHE ou de la position ARRÊT à la position de commutation.

11. Système d'accouplement chirurgical selon la revendication 10, **caractérisé en ce que** le dispositif de déblocage (294) est réalisé de manière à ce que l'organe de déblocage (120), dans la position actionnée, transfère l'élément de commande (212) de la position d'entrainement à la position de dégagement pour empêcher que l'organe de déblocage (120) puisse être amené directement ou indirectement en contact ou en prise avec le troisième élément de verrouillage (286) pour transférer le dispositif de verrouillage (288) de la position MARCHE ou de la position ARRÊT à la position de commutation.

12. Système d'accouplement chirurgical selon la revendication 10 ou la revendication 11, **caractérisé en ce que** le dispositif de déblocage (294) est réalisé de manière telle, que l'élément de commande (212) puisse être transféré de la position de dégagement à la position d'entraînement, uniquement par transfert de l'organe de déblocage (120) de la position actionnée à la position non actionnée.

13. Système d'accouplement chirurgical selon l'une des revendications 10 à 12, **caractérisé en ce que** le dispositif de déblocage (294) est réalisé de manière à ce que l'organe de déblocage (120) puisse, uniquement dans la position d'entraînement, être amené directement ou indirectement en contact ou en prise avec le troisième élément de verrouillage (286) pour le transfert du dispositif de verrouillage (288) de la position MARCHE ou de la position ARRÊT à la position de commutation.

14. Système d'accouplement chirurgical selon l'une des revendications 10 à 13, **caractérisé en ce que** le dispositif de déblocage (294) comprend un dispositif de rappel (220) pour le transfert automatique de l'élément de commande (212) de la position de dégagement à la position d'entraînement, dès que l'organe de déblocage (120) est transféré de la position actionnée à la position non actionnée.

15. Système d'entraînement chirurgical (10) comprenant au moins une pièce formant poignée (14) avec un entraînement sous la forme d'un moteur électrique (82) et au moins un conducteur d'alimentation électrique (20), ce conducteur d'alimentation électrique (20) présentant une première extrémité qui peut être reliée de manière amovible à ladite au moins une pièce formant poignée (14), et une deuxième extrémité reliée ou pouvant être reliée de manière amovible à un dispositif de commande et/ou de régulation (12) pour la commande et/ou la régulation du moteur électrique (82), **caractérisé par** un système d'accouplement (60) pour assurer la liaison électrique et mécanique de la pièce formant poignée (14) et du conducteur d'alimentation (20), selon l'une des revendications précédentes.
